# EUROPEAN PATENT APPLICATION

(11) **EP 3 960 859 A1**
(43) Date of publication of application: **02.03.2022**
(21) Application number: 20755751.3
(22) Date of filing: 14.02.2020
(51) Int. Cl.: C12N 15/113, A61K 31/7105, A61K 47/00

(54) **NANOSYSTEM BASED ON MICRORNA FOR TREATING OBSESITY**

(30) Priority: 15.02.2019 ES 201930118
(71) Applicant: Servicio Andaluz de Salud, 41071 Sevilla (ES); Universidad De Malaga, E-29071 Málaga (ES); Universidad de Sevilla, 41013 Sevilla (ES); Ciber, 28029 Madrid (ES)
(72) Inventor: LHAMYANI, Said, 29010 Málaga (ES); MARIEL GENTILE, Adriana, 29010 Málaga (ES); TINAHONES MADUEÑO, Francisco José, 29010 Málaga (ES); EL BEKAY RIZKY, Rajaa, 29010 Málaga (ES); GIRÁLDEZ PÉREZ, Rosa María, 29590 Málaga (ES); GRUESO MOLINA, Elia María, 41013 Sevilla (ES); PÉREZ TEJEDA, Mª Pilar, 41013 Sevilla (ES)
(74) Representative: Hoffmann Eitle
(86) International application number: PCT/ES2020/070104
(87) International publication number: WO 2020/165482

(57) **Abstract**

The present invention relates to compositions, methods and uses, consisting of a functionalised miRNA-based nanosystem for treating obesity, weight loss and/or reduction of localised fat, which comprises miR-21 or other derived or equivalent compounds such as modified polynucleotides, synthetic mimetic and/or isomiR of miR-21; and a carrier which comprises an optimised nanoparticle for effectively binding oligonucleotides, forming an adequate nanosystem for *in vivo* transfection and, in particular, for the *in vivo* release of genes in fatty tissue.

## Description

The present invention relates to the field of precision medicine, more specifically to the field of gene therapy, and particularly to the field of microRNA (miRNA) therapy. The present invention describes the therapeutic use of a nanosystem comprising the microRNA-21 (miR-21) molecule or an equivalent molecule (modified polynucleotides derived from same, mimetic, isomiR) and a nanoparticle-based carrier for obesity and diseases related to weight loss and/or fat reduction.

### PRIOR STATE OF THE ART

Today, obesity is recognised as a chronic disease caused, from a general point of view, by an energy imbalance between calories consumed and calories burned. The prevalence of this disease has increased drastically over the past few decades with a clear upward trend in the near and distant future, so much so that it is considered one of the most serious health problems plaguing society today. The increase in comorbidities (simultaneous chronic diseases) related to obesity has made the medical community more aware of obesity as a disease with life-threatening implications. These associated health risks include heart diseases, strokes, hypertension, type 2 diabetes mellitus, dyslipidaemia, sleep apnoea, osteoarthritis, gallbladder diseases, depression and certain forms of cancer (for example, endometrial cancer, breast cancer, prostate cancer and colon cancer).

The overall level of unmet needs on the market of medicinal products for obesity is extremely high, with inefficiencies at all levels in the payer-prescriber-patient relationship, which represents a good opportunity for the pharmaceutical and medical device industries to provide solutions. The current range of pharmacological interventions is affected by a lack of options, and the available medicinal products only offer a moderate efficacy and have problematic side effect profiles. The few therapy options are Xenical (orlistat), which is the only pharmacological agent approved on the seven main markets, Canada and Brazil, together with phentermine, Qsymia and Belviq, that are only available in the United States. There has been a serious lack of novel therapies because pharmaceutical companies are reluctant to face the challenges that may arise in obtaining regulatory approval. In countries other than the United States, doctors only have Xenical. More commonly, they resort to medicinal products indicated for other conditions and this does not ensure minimal effectiveness or safety for the patients, causing unnecessary side effects of that product. This is a highly neglected market which needs innovative medicinal products and devices to fill the void.

Treatment for obesity presents many difficulties in daily clinical practice due to its very high rate of failures. Although weight loss is initially achieved, patients recover the lost weight in the medium and long terms. Most of the existing strategies for controlling obesity are based on establishing a negative energy balance by decreasing intake and/or reducing energy absorption. However, genetic, metabolic and neuroendocrine factors seem to influence the pathogenesis of obesity and make weight loss a much more complex process.

Several physiological systems are involved in body weight and energy homeostasis regulation, and these systems are interrelated through several complex feedback mechanisms. Although this complexity gives rise to a multitude of therapeutic targets treatable with pharmacotherapy, interregulation also causes the development of an effective pharmacological treatment for obesity and overweight to be difficult and require investing a great deal of time in research.

Furthermore, once a pharmacological agent generally effective for treating obesity and/or overweight has been discovered, the genetic and/or physiological differences between individuals can give rise to significant variations in efficacy among a population of patients; i.e., there is a tendency for subpopulations of patients to respond or not respond to a therapeutic agent based on the physiology of the individual.

Although knowledge of this pathology has advanced by leaps and bounds, it is obvious that current knowledge is not sufficient to mitigate this epidemic, and there is an imperative need to conduct research on new therapies in order to control and/or prevent said pathology. Current strategies against obesity aim at restricting energy absorption, but treatment for obesity is still far from being satisfactory.

It would be useful to provide a new therapeutic approach that would act, perhaps at the gene level, on a general cellular mechanism that is shared within one or more cell groups that play a crucial role, such as adipocytes, such that said mechanism would be a common mechanism regardless of the individual, and the possibility of obtaining effective treatment would thereby be simplified.

### Browning:

Until the end of the 1980s, adipose tissue had been considered a simple storage organ; however, evidence showed adipose tissue to be an endocrine organ that secretes many hormones and cytokines which can influence systemic metabolism. Furthermore, the existence of different fat depots playing specific roles has been established. The main fatty tissue is white adipose tissue (WAT) which stores mainly energy and increases in obesity; on the other hand, there is brown adipose tissue (BAT) which regulates temperature by means of producing heat through a mechanism called thermogenesis which increases energy consumption. A third type emerges when brown adipocytes appear in anatomically characteristic sites of WAT, in a process called WAT browning, hereinafter simply browning, such that these brown adipocytes emerging in WAT emerge from precursor cells that are different from those of classical brown adipocytes and are closer to the white adipocyte lineage, and they are often called "inducible, beige or brite". Brown and beige fats have been considered a target for controlling obesity.

Morphologically, WAT forms unilocular adipocytes containing a single lipid droplet or vacuole storing excess energy in the form of triglycerides and is widely distributed in most organs as subcutaneous adipose tissue (SAT).

On the other hand, BAT forms multilocular adipocytes made up of small lipid droplets and a large number of mitochondria. Furthermore, it is a highly vascularised tissue that is densely innervated by the sympathetic nervous system characterised by producing a high expression level of uncoupling protein 1 (UCP1) in the internal mitochondrial membrane. BAT is mainly localised in interscapular fat depots (int. SAT), supraclavicular fat depots, adrenal fat depots, pericardial fat depots, para-aortic fat depots, fat depots around the pancreas, inguinal fat depots (ISAT), kidneys and trachea.

In brown adipocytes, UCP1 plays a critical role to allow the release, instead of the storage, of electrons, which leads to the release of heat. Brown adipocytes can dissipate the chemical energy stored as triglycerides by channelling fatty acids into β oxidation when they are activated. UCP1 is capable of uncoupling electron transport in adenosine triphosphate (ATP) production and produces heat in brown adipocytes.

Beige adipose tissue is a tissue similar to brown adipose tissue formed by multilocular adipocytes with several lipid droplets, a high mitochondrial content and the expression of several genes specific to brown adipose tissue (UCP1, Cidea). Under baseline conditions, beige adipocytes exhibit a low thermogenic activity and a reduced expression of UCP1 compared with brown adipocytes.

Browning is known to occur after a thermogenic stimulus, such as a prolonged exposure to cold, or can be mimicked with a chronic treatment using β-3 adrenergic receptor activators.

In addition to thermoregulating genes, such as UCP-1, beige adipocytes express other cell surface marker genes, such as CD137 and transmembrane protein 26 (Tmem 26). Recent studies revealed that the activity of beige adipose tissue is negatively associated with body mass index and/or body fat. Other findings showed that brown and beige adipose tissue activity stimulation improves glucose tolerance and insulin sensitivity. This data suggests that the induction of the browning process and/or recruitment of brown adipose tissue in human subjects can be a therapeutic target for obesity and insulin resistance. Other studies showed that mice having less brown adipose tissue (by means of genetic engineering) or being UCP 1-deficient are more susceptible to gaining weight and developing obesity compared with normal mice.

Browning is well defined up to this point; however, compounds which allow effectively inducing the production of beige fat in WAT are still not available. Accordingly, there are no effective treatments on the market for obesity based on the strategy of increasing the amount and/or activity of beige fat.

### MicroRNAs:

It is known in the state of the art that microRNAs are involved in all important cellular processes, regulating gene expression at the post-transcription level. Currently, miRNAs are well known as a class of biological macromolecules consisting of small, naturally occurring, non-coding RNA molecules of about 22 nucleotides which regulate gene expression at the post-transcription level in eukaryotes and therefore participate in a wide range of biological processes. Their main function is to regulate gene expression, and they are present in all human cells. Currently, the important contribution of miRNAs in the organisation and commissioning of a regulatory programme for gene expression and how these molecules can alter this regulation and cause diseases in the event of being overexpressed or underexpressed are known. Several studies show various miRNAs playing vital functions in controlling glucose homeostasis, in insulin pathways in the development and maintenance of pancreatic islets, etc. Other studies show that specific miRNAs intervene during adipocyte differentiation and maturation, thereby contributing to the development and/or control of obesity, and also intervening in the development of insulin resistance. Various studies showed that the expression of miRNAs in pre-adipocytes is altered during the development of fat cells and obesity.

However, there is a need to conduct specific studies focusing on understanding the role of miRNAs in adipocyte proliferation and differentiation during the development of fat cells, as well as on transcriptional regulation for regulating metabolic homeostasis, which allows identifying new therapeutic targets for obesity, and therefore the associated metabolic diseases thereof.

In recent years, miRNAs have therefore emerged as a therapeutic reagent capable of acting at the post-transcription regulatory level and with a different approach to medicinal products used today for diseases such as obesity and/or overweight. However, the use of therapeutic reagents of this type presents new technical problems that must be addressed so that they may represent a real and improved alternative responding to the main challenges in the field of human health. These technical problems include:
- Identifying miRNAs with effective biological activity,
- Finding dosing compositions that ensure the functional stability of these macromolecules.
- Devising delivery methods for transferring miRNAs into the desired tissue, preventing accumulation and side effects in other tissues.
- Designing compositions that ensure high cell transfection.

A major challenge would be to develop delivery methods for transferring miRNAs or their inhibitors into the desired tissue, without incurring in bioaccumulation in tissues that will lead to toxicity and unwanted side effects. In this aspect, some metabolic tissues may be more accessible than others. The administration system for administering drugs and achieving therapeutic effects is currently a field of interest in the treatment of many diseases, such as cancer.

However, the development of therapies for the administration of microRNAs still poses a major challenge.

### DESCRIPTION OF THE INVENTION

The authors of the present invention have developed compositions, methods and uses consisting of a functionalised miRNA-based nanosystem for treating obesity, weight loss and/or reduction of localised fat, which comprises miR-21 or other derived or equivalent compounds such as modified polynucleotides, synthetic mimetic and/or isomiR of miR-21; and a carrier which comprises an optimised nanoparticle for effectively binding oligonucleotides, forming an adequate nanosystem for *in vivo* transfection and, in particular, for the *in vivo* release of genes in fatty tissue.

As part of the disclosure, the inventors have demonstrated by means of experiments that the miR-21-based functionalised nanosystem selectively promotes energy expenditure by means of inducing browning, as can be verified in Example 4. It has therefore been proven that this miRNA and equivalents are of great interest as therapeutic agents for anti-obesity treatments and that their inclusion in a carrier optimised with nanoparticles, forming high-efficiency nanosystems, increases the body's capacity to metabolise large amounts of glucose and lipids measured by brown and beige fats in proportion to the tissue mass thereof. Furthermore, the experiments conducted also show that the developed nanosystems enhance the capacity of miR-21 to produce positive effects for metabolic disturbances associated with dysfunctional white adipose tissue accumulation. This solution has the advantage that miR-21 can selectively promote energy expenditure by means of inducing the browning of white adipose tissue, therefore converting white fat to beige fat and inducing the body to metabolise large amounts of glucose and lipids in proportion to the adipose tissue mass thereof in an effective and irreversible manner. Likewise, since it is known that obesity-associated adipose tissue dysfunction leads to the onset of metabolic disturbances, the nanosystem designed by the inventors is of therapeutic interest for the treatment of metabolic diseases such as: amyloidosis, cardiometabolic disease, dehydration, diabetes (type 1, type 2, diabetic foot ulcers, diabetic macular oedema, diabetic neuropathy, diabetic retinopathy, diabetic nephropathy, gestational diabetes, dyslipidaemia, hyperlipidaemia), glucose intolerance, hypercholesterolaemia, hyperglycaemia, hyperinsulinaemia, or insulin resistance, hyperkalaemia, hypoglycaemia, hypopotassaemia, lipodystrophy (lipoatrophy), metabolic syndrome, obesity, overweight, osteopenia, osteoporosis (including postmenopausal osteoporosis), phenylketonuria (PKU), hypersecretion of pituitary ACTH (Cushing's syndrome) and Pompe disease.

Therefore, a first aspect of the invention relates to a functionalised nanosystem for transporting biologically active molecules, hereinafter nanosystem of the invention, comprising:
- A biologically active molecule which is selected from an miR-21 microRNA, mimetic, isomiR, oligonucleotide molecule with more than 75% sequence similarity, compounds aimed at increasing the transcription or activity of miR-21 or a source thereof, or any of the combinations thereof.
- A carrier suitable for transporting biologically active molecules comprising a nanoparticle and a transporter molecule, capable of linking oligonucleotides.

The expression "biologically active molecule" is in a broad sense and comprises molecules such as drugs having a high or more preferably low molecular weight, polysaccharides, proteins, peptides, lipids, oligonucleotides and nucleic acids, as well as combinations thereof. In a variant of the invention, the function of the biologically active molecule is to prevent, mitigate, cure or diagnose diseases. In another variant of the invention, the biologically active molecule has an aesthetic, cosmetic and/or veterinary function.

Similarly, in this specification, the expression "biologically active molecule" also includes the terms "active ingredient", "active substance", "pharmaceutically active substance", "therapeutic agent", "drug" or "pharmaceutically active ingredient", i.e., it means any component potentially providing a pharmacological activity or another different effect in the diagnosis, cure, mitigation, alleviation, prevention or treatment of a disease or affecting the structure or function of the body of humans or other animals. It also includes "cosmetic" or "aesthetic" components. The term also includes those components that promote a chemical change in drug production and are present therein in an expected modified form providing the specific activity or effect.

In this specification, "miRNA mimetics" are understood to mean molecules designed to imitate the function of a specific miRNA, such that they contain non-natural or artificial, double-stranded miRNA-type RNA fragments. These RNA fragments are constructed to contain a sequence motif conferring them a functionality similar to the functionality of the miRNA which they imitate. In this specification, these mimetics refer to those miRNA containing a percentage of identity of the functional region of a specific miRNA of at least 70%, or more preferably of at least 80%, or more preferably of 90%. Once these RNA fragments are introduced in cells, the miRNA mimetics fulfil a function equivalent to the imitated miRNA. The result is the post-transcription repression or activation of specific genes, which allows specific approaches for studying the function of genes useful in the treatment of diseases.

The term "carrier", in a broad sense, refers to an element that binds to and facilitates the transport of biologically active molecules. It can comprise more than one element in its structure and at least one of these elements has the property of readily binding to the biologically active molecule, preferably to oligonucleotides, and this is called a "transporter molecule", and they can be, but without limitation, a polypeptide, protein, lipid or molecule with at least one polymeric chain.

Similarly, in this specification, the term "carrier" also includes any compound which, combined with a biologically active molecule, improves the functional stability of the biologically active molecule, conferring the biologically active molecule greater capacity to reach the desired tissue where it potentially provides a pharmacological activity or provides the biologically active molecule with high cell transfection.

Preferably, but without limitation, the biologically active molecule and the nanoparticle are bound to the transporter molecule independently. This bond can be either a covalent bond or a non-covalent bond. Preferably, it will be a non-covalent bond which generally takes place by means of electrostatic interactions, hydrophobic interactions, surface adsorption, encapsulation or intercalated inside same.

The term "nanoparticle" refers to any particle between 1 and 20 nm which can be made of any known nanomaterial such as, for example, but without limitation, nanoparticles based on carbon, silica, chitosans, metals (gold, silver, iron or oxides thereof), liposomes, niosomes, dendrimers or composite materials of those previously mentioned.

In another preferred embodiment, the nanosystem is made up of a nanoparticle having a cationic gold metal core which allows for the composition not to accumulate in tissues and not to generate toxicity in the body of humans or animals. Furthermore, gold nanoparticles confer greater stability to the nanosystem than other metals such as silver, due to their lower aggregation tendency, which allows a high cell absorption. Preferably, the mean nanoparticle size is comprised between 3 and 7 nanometres.

In an additional preferable embodiment, the nanosystem according to any of the preceding embodiments is made up of a transporter molecule which is a cationic surfactant. More preferably, the cationic surfactant is a Gemini surfactant which provides greater capacity for complexing oligonucleotides and for which a high affinity for adipose tissue has been determined. The experiments conducted by the inventors shown in Example 2 show that Gemini surfactants 16-3-16 and 16-Ph-16 exhibit particularly notable characteristics for complexing oligonucleotides, which allows for a more compact nanosystem providing better cell transfection of the nanosystem and having a high affinity for adipose tissue. Additionally, these surfactants ensure a high biodegradability and biocompatibility which, along with their high efficacy, ensure that the nanosystem does not accumulate in tissues and generate toxicity in the body of humans or animals.

In this specification, the term "Gemini surfactant" refers to a group of dimeric surface active agents with two hydrophobic or non-polar tails and two hydrophobic or polar groups with a spacer binding the two polar groups in a rigid or flexible manner, schematically depicted as m-s-m (where "m" is the hydrophobic tails and "s" the spacer). The polar groups may have a positive charge (ammonium) or a negative charge (phosphate, sulfate, carboxylate), whereas non-ionic polar groups can be polyethers or sugars. It can incorporate in its structure peptidic, glucidic, or mainly lipidic components. The central spacer molecule can be tailored to the needs of the invention by binding two or more molecules of interest. To minimise potential toxicity problems with these compounds, the structure of spacer molecules can be made up of α-amino acids, carbohydrates, spermine, etc., which are furthermore biodegradable. The polar groups are mainly ammoniums, although they may be included with more complex structures, such as amino acids.

Taking the aforementioned features into account, the present invention seeks to combine the greater efficiency of nanoparticles in terms of their absorption into/elimination from the cell interior, as well as the protection of biomolecules such as RNA from enzymatic degradation, with the low toxicity and the greater biomolecule compaction-inducing capacity of cationic Gemini surfactants, thereby constructing new, more efficient vectors having a low toxicity. However, studies of the synthesis of nanosystems based on said surfactants which complex biomolecules such as DNA or RNA have been non-existent until now, hence the novelty of the synthesised system. To successfully carry out the synthesis process, gold nanoparticles functionalised with biodegradable Gemini surfactants, also called precursors 16-3-16 and 16-Ph-16, will first be prepared.

Furthermore, since these surfactants have been initially designed to act as possible detergents, they are compounds potentially related to fatty tissue in which the biologically active molecules are capable of inducing the browning of the fatty tissue. All this allows the amount of miRNA required for acting on the target tissue to be considerably reduced while being, at the same time, a highly selective device, preventing unwanted adverse effects in tissues. Therefore, the functionalised nanosystem of the invention focuses the action of the biologically active miRNA molecule, making it possible to control material unloading in the target tissue, providing it with ideal properties for use in the treatment of obesity.

In a particular embodiment of the first aspect of the invention, the mean size of the nanoparticle in a nanosystem which is made up of Gemini surfactant 16-3-16, the mean size of the nanoparticles would be comprised between 3 and 4 nanometres; whereas for a nanosystem which is made up of Gemini surfactant 16-ph-16, the mean size of the nanoparticles would be comprised between 5 and 6 nanometres.

In another particular embodiment, the nanosystem according to any of the preceding embodiments has a mean size comprised between 30 and 80 nanometres. More preferably, in a nanosystem which is made up of Gemini surfactant 16-3-16, the mean size of the nanosystem would be comprised between 40 and 60 nanometres; whereas for a nanosystem which is made up of Gemini surfactant 16-ph-16, the mean size of the nanosystem would be comprised between 50 and 70 nanometres.

According to the preceding particular embodiments, the authors of the present invention have verified that, following administration in the body of humans or other animals, the nanosystem can be well eliminated from the body, once the biologically active molecule has performed its function. This capacity is directly related to the small size of the gold core of the nanosystem and its high positive charge conferring additional stability to the system.

A second aspect of the invention relates to a composition comprising at least one nanosystem of the invention, hereinafter composition of the invention. This composition will preferably be a pharmaceutical composition.

A third aspect of the invention relates to the nanosystem of the invention or the composition of the invention for use as a medicinal product.

A fourth aspect of the invention relates to the nanosystem of the invention or the composition of the invention for preventing, delaying, mitigating, reversing, curing and/or treating a metabolic disease. More preferably, the metabolic disease is selected from the list consisting of: amyloidosis, cardiometabolic disease, dehydration, diabetes (type 1, type 2, diabetic foot ulcers, diabetic macular oedema, diabetic neuropathy, diabetic retinopathy, diabetic nephropathy, gestational diabetes, dyslipidaemia, hyperlipidaemia), glucose intolerance, hypercholesterolaemia, hyperglycaemia, hyperinsulinaemia, or insulin resistance, hyperkalaemia, hypoglycaemia, hypopotassaemia, lipodystrophy (lipoatrophy), metabolic syndrome, obesity, overweight, osteopenia, osteoporosis (including postmenopausal osteoporosis), phenylketonuria (PKU), hypersecretion of pituitary ACTH (Cushing's syndrome) and Pompe disease.

In a preferred embodiment, the metabolic disease is selected from obesity, overweight and hyperinsulinaemia.

A fifth aspect of the invention relates to the nanosystem of the invention or the composition of the invention for preventing, delaying, mitigating, reversing, curing and/or treating a disease associated with body weight gain. More preferably, the disease associated with body weight gain is selected from the list consisting of hypothyroidism, Cushing's syndrome, hypogonadism, hypothalamic lesions, growth hormone deficiency, Prader-Willi syndrome, Bardet-Biedl syndrome, Cohen syndrome, MOMO syndrome, anxiety and depression; or any of the combinations thereof.

A sixth aspect of the invention provides a kit-of-parts, hereinafter kit-of-parts of the invention, comprising among its components (a) the nanosystem of the invention or the composition of the invention; and a medicinal product which is selected from:
(b1) a medicinal product associated with weight gain which is selected from the list consisting of: atenolol, carbamazepine, citalopram, clozapine, doxazosin mesylate, doxepin, escitalopram, fluvoxamine, gabapentin, gamma-hydroxybutyric acid, leuprolide, lithium, metoprolol, mirtazapine, nateglinide, nortriptyline, olanzapine, paroxetine, pioglitazone, propranolol, quetiapine, repaglinide, risperidone, terazosin, valproate and phenytoin; or
(b2) a medicinal product for obesity approved in a national agency which is selected from the list consisting of: megestrol acetate, benzphetamine, caffeine, cathinone, cetilistat, clobenzorex, chlorphentermine hydrochloride, dexfenfluramine hydrochloride, diethylpropion hydrochloride, fenfluramine hydrochloride, phenmetrazine hydrochloride, phentermine hydrochloride, lorcaserin hydrochloride, mefenorex hydrochloride, sibutramine hydrochloride, dronabinol, phendimetrazine, fenfluramine, phenylpropanolamine, fenproporex, phentermine, fluoxetine, levocarnitine, levothyroxine sodium, mazindol, methamphetamine, methylcellulose, orlistat, phendimetrazine, rimonabant, Saxenda, sibutramine, amphetamine sulfate, phendimetrazine tartrate, "bupropion hydrochloride + naltrexone", "phentermine hydrochloride + topiramate", "levocarnitine + sibutramine" and "metformin + sibutramine"; or
(b3) a medicinal product for obesity in the clinical development phase which is selected from the list consisting of: Adipotide, AKR-001, AM-833, AMG-598, beloranib, BI-456906, biotin, betahistine hydrochloride, lorcaserin hydrochloride, lorcaserin hydrochloride, efpeglenatide, G-3215, GMA-102, GT-001, GTS-21, HM-12525A, HM-15211, HSG-4112, LLF-580, MEDI-0382, MET-2, Miricorilant, NGM-313, NGM-386, NN-9277, NN-9423, NN-9536, NNC-01651562, NNC-01651875, Novdb-2, NovOB, pegapamodutide, REGN-4461, RZL-12, S-237648, S-237648, SAR-425899, SCO-792, setmelanotide, setmelanotide, tesofensine, TP-0101, VP-01, ZGN-1061, ZP-4982, (acarbose + orlistat), (leucine + sildenafil citrate), (leucine + metformin hydrochloride + sildenafil citrate) and (metoprolol + tesofensine).

A seventh aspect of the invention relates to the kit-of-parts of the invention for preventing, delaying, mitigating, reversing, curing and/or treating a metabolic disease. More preferably, the metabolic disease is selected from the list consisting of: amyloidosis, cardiometabolic disease, dehydration, diabetes (type 1, type 2, diabetic foot ulcers, diabetic macular oedema, diabetic neuropathy, diabetic retinopathy, diabetic nephropathy, gestational diabetes, dyslipidaemia, hyperlipidaemia), glucose intolerance, hypercholesterolaemia, hyperglycaemia, hyperinsulinaemia, or insulin resistance, hyperkalaemia, hypoglycaemia, hypopotassaemia, lipodystrophy (lipoatrophy), metabolic syndrome, obesity, overweight, osteopenia, osteoporosis (including postmenopausal osteoporosis), phenylketonuria (PKU), hypersecretion of pituitary ACTH (Cushing's syndrome) and Pompe disease.

In a preferred embodiment, the metabolic disease is selected from obesity, overweight and hyperinsulinaemia.

A final aspect relates to a method for synthesising the nanosystem of the invention, hereinafter method of the invention, which comprises:
(a) adding a Gemini surfactant as a stabilising agent in a solution of hydrogen tetrachloroaurate tetrahydrate (HAuCl₄•4H₂O),
(b) reducing hydrogen tetrachloroaurate tetrahydrate (HAuCl₄•4H₂O) by means of the controlled addition of a reducing agent, and
(c) complexing with the biologically active molecule by means of the controlled addition of the miR-21 microRNA, mimetic, isomiR, oligonucleotide molecule with more than 75% sequence similarity, compounds aimed at increasing the transcription or activity of miR-21 or a source thereof, or any of the combinations thereof; with excess polymer and stirring conditions being maintained."

Preferably, reducing hydrogen tetrachloroaurate tetrahydrate which is performed in step (b) of the method of the invention is performed by means of using a sodium borohydride solution (NaBH₄) as a reducing agent. More preferably, the NaBH₄ is at a known concentration of between 0.1 and 0.2 M; and even more preferably with a dropwise fractionated dosing.

In another preferred embodiment, a step of vigorous stirring is performed between step (a) and step (b) of the method of the invention. Preferably, this step is performed in the absence of light. More preferably, this vigorous stirring takes place for at least 3 minutes, even more preferably for at least 4 minutes, and much more preferably for at least 5 minutes.

In another preferred embodiment, a step of stirring is performed between step (b) and step (c) of the method of the invention. Preferably, this step is performed in the absence of light. More preferably, this stirring takes place for at least 10 minutes, even more preferably for at least 13 minutes, and much more preferably for at least 15 minutes.

In another preferred embodiment, a step of gentle stirring is performed after step (c) of the method of the invention. Preferably, this step is performed for at least 20 minutes, more preferably for at least 25 minutes, and even more preferably for at least 30 minutes.

In an additional preferred embodiment, the concentration of Gemini surfactant, [T], of steps (a) and (b) of the method of the invention will be a specific concentration so as to obtain a nanosystem having optimal size and stability. This concentration, [T], will be directly related to the critical micelle concentration, cmc, of the surfactant, where the ratio between them is equivalent to [T]/cmc less than 20 and greater than 1. Preferably, this ratio will be [T]/cmc less than 7 and greater than 3. More preferably, this ratio will be [T]/cmc equal to 5.

Throughout the description and claims, the word "comprises" and variants thereof do not mean to exclude other technical features, additions, components or steps. Furthermore, the word "comprises" includes the case "consists of". For those skilled in the art, other objects, advantages and features of the invention will be deduced in part from the description and in part from putting the invention into practice. The following examples and drawings are provided by way of illustration and are not meant to restrict the present invention. Numerical signs relative to the drawings and placed between parentheses in a claim are solely for the purpose of better understanding the claim and must not be interpreted as limiting the scope of protection of the claim. Furthermore, the present invention covers all the possible combinations of particular and preferred embodiments herein indicated.

### BRIEF DESCRIPTION OF THE FIGURES

Figure 1A shows the UV-vis spectrum for precursor Au@16-Ph-16.
Figure 1B shows the UV-vis spectrum for precursor Au@16-3-16.
Figure 1C shows the UV-vis spectrum for functionalised nanosystem Au@16-Ph-16/miR-21, R = 0.124.
Figure 1D shows the UV-vis spectrum for functionalised nanosystem Au@16-3-16/miR-21, R = 0.378.
Figure 1E shows the UV-vis spectrum for functionalised nanosystem Au@16-Ph-16/miR-21, R = 0.093.
Figure 1F shows UV-vis spectrum for functionalised nanosystem Au@16-3-16/miR-21, R = 0.283.
Figure 1G shows UV-vis spectrum for functionalised nanosystem Au@16-Ph-16/miR-21, R = 0.062.
Figure 1H shows the UV-vis spectrum for functionalised nanosystem Au@16-3-16/miR-21, R = 0.189.
Figure 2 shows TEM images corresponding to the synthesis of nanoparticles coated with cationic Gemini surfactant: (A) Au@16-Ph-16 and (B) Au@16-3-16.
Figure 3 shows AFM topography images of Au@16-3-16/miR-21 adsorbed on APTES-modified mica, at different ratios of R. (A-B) R = 0.378; (C-D) R = 0.283; (E-F) R = 0.189. Figures B, D and F correspond to the transverse analysis of heights along the indicated line corresponding to images A, C and E, respectively.
Figure 4 shows AFM topography images of Au@16-Ph-16/miR-21 adsorbed on APTES-modified mica, at different ratios of R. (A-B) R = 0.124; (C-D) R = 0.093; (E-F) R = 0.062. Figures B, D and F correspond to the transverse analysis of heights along the indicated line corresponding to images A, C and E, respectively.
Figure 5A shows the formation and stability of the nanosystem Au@16-pH-16 *in situ.*
Figure 5B shows the formation and stability of the nanosystem Au@16-pH-16 at 24 hours.
Figure 5C shows the formation and stability of the nanosystem Au@16-pH-16 at 48 hours.
Figure 5D shows the formation and stability of the nanosystem Au@16-pH-16 at 1 week.
Figure 5E shows the formation and stability of the nanosystem Au@16-pH-16 at 2 weeks.
Figure 5F shows the formation and stability of the nanosystem Au@16-pH-16 at 1 month.
Figure 5G shows the formation and stability of the nanosystem Au@16-pH-16/miR-21, R = 0.124, *in situ.*
Figure 5H shows the formation and stability of the nanosystem Au@16-pH-16/miR-21, R = 0.124, at 24 hours.
Figure 5l shows the formation and stability of the nanosystem Au@16-pH-16/miR-21, R = 0.124, at 48 hours.

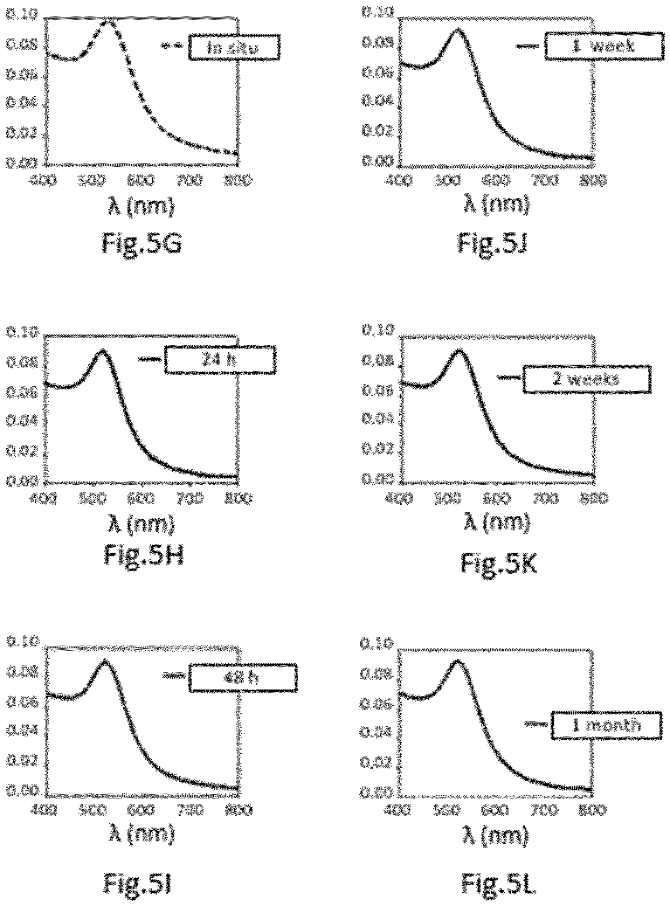

Figure 5J shows the formation and stability of the nanosystem Au@16-pH-16/miR-21, R = 0.124, at 1 week.
Figure 5K shows the formation and stability of the nanosystem Au@16-pH-16/miR-21, R = 0.124, at 2 weeks.
Figure 5L shows the formation and stability of the nanosystem Au@16-pH-16/miR-21, R = 0.124, at 1 month.

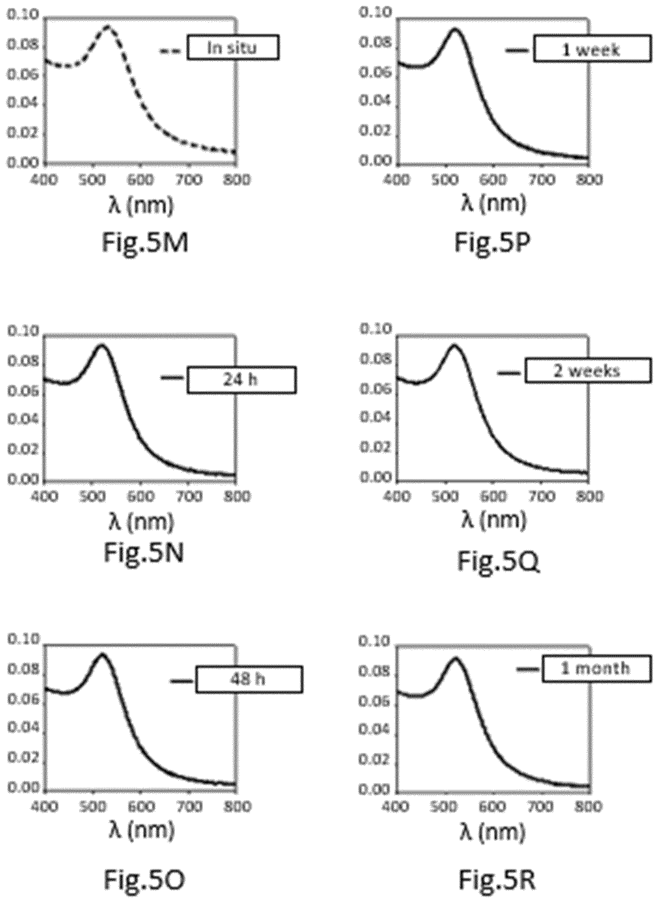

Figure 5M shows the formation and stability of the nanosystem Au@16-pH-16/miR-21, R = 0.093, *in situ.*
Figure 5N shows the formation and stability of the nanosystem Au@16-pH-16/miR-21, R = 0.093, at 24 hours.
Figure 5O shows the formation and stability of the nanosystem Au@16-pH-16/miR-21, R = 0.093, at 48 hours.
Figure 5P shows the formation and stability of the nanosystem Au@16-pH-16/miR-21, R = 0.093, at 1 week.
Figure 5Q shows the formation and stability of the nanosystem Au@16-pH-16/miR-21, R = 0.093, at 2 weeks.
Figure 5R shows the formation and stability of the nanosystem Au@16-pH-16/miR-21, R = 0.093, at 1 month.

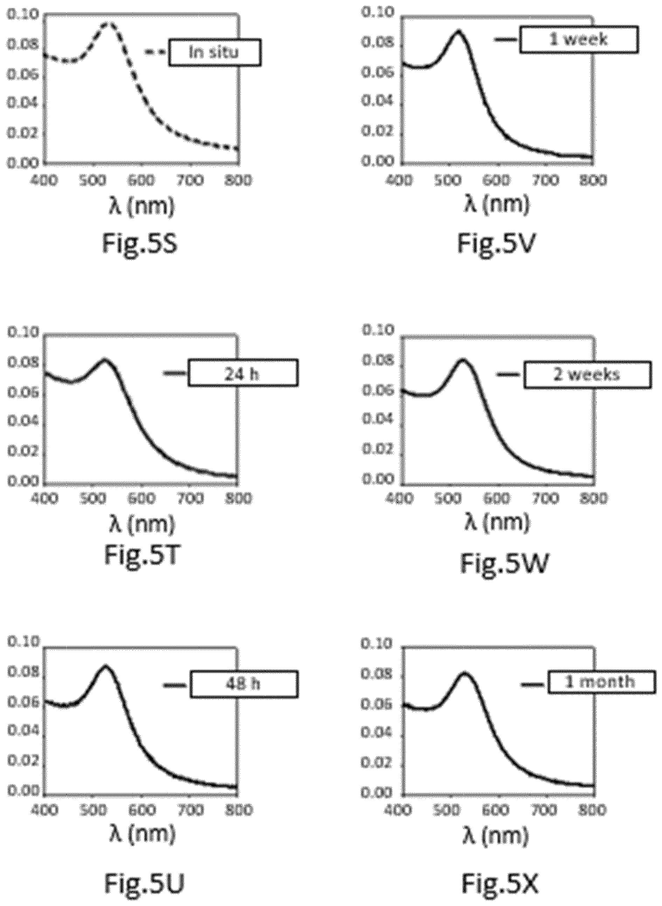

Figure 5S shows the formation and stability of the nanosystem Au@16-pH-16/miR-21, R = 0.063, *in situ.*
Figure 5T shows the formation and stability of the nanosystem Au@16-pH-16/miR-21, R = 0.063, at 24 hours.
Figure 5U shows the formation and stability of the nanosystem Au@16-pH-16/miR-21, R = 0.063, at 48 hours.
Figure 5V shows the formation and stability of the nanosystem Au@16-pH-16/miR-21, R = 0.063, at 1 week.
Figure 5W shows the formation and stability of the nanosystem Au@16-pH-16/miR-21, R = 0.063, at 2 weeks.
Figure 5X shows the formation and stability of the nanosystem Au@16-pH-16/miR-21, R = 0.063, at 1 month.

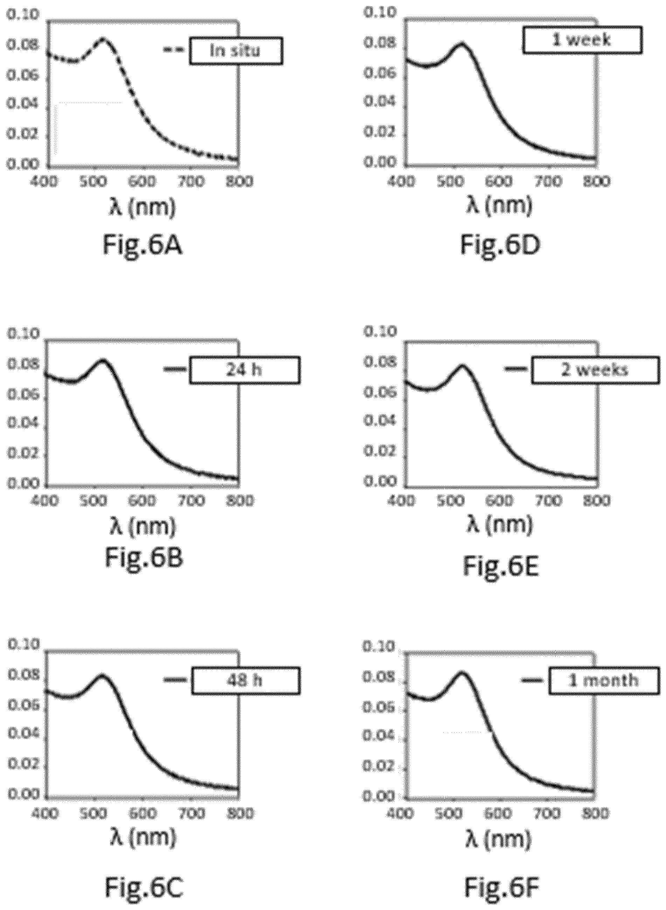

Figure 6A shows the formation and stability of the nanosystem Au@16-3-16 *in situ.*
Figure 6B shows the formation and stability of the nanosystem Au@16-3-16 at 24 hours.
Figure 6C shows the formation and stability of the nanosystem Au@16-3-16 at 48 hours.
Figure 6D shows the formation and stability of the nanosystem Au@16-3-16 at 1 week.
Figure 6E shows the formation and stability of the nanosystem Au@16-3-16 at 2 weeks.
Figure 6F shows the formation and stability of the nanosystem Au@16-3-16 at 1 month.

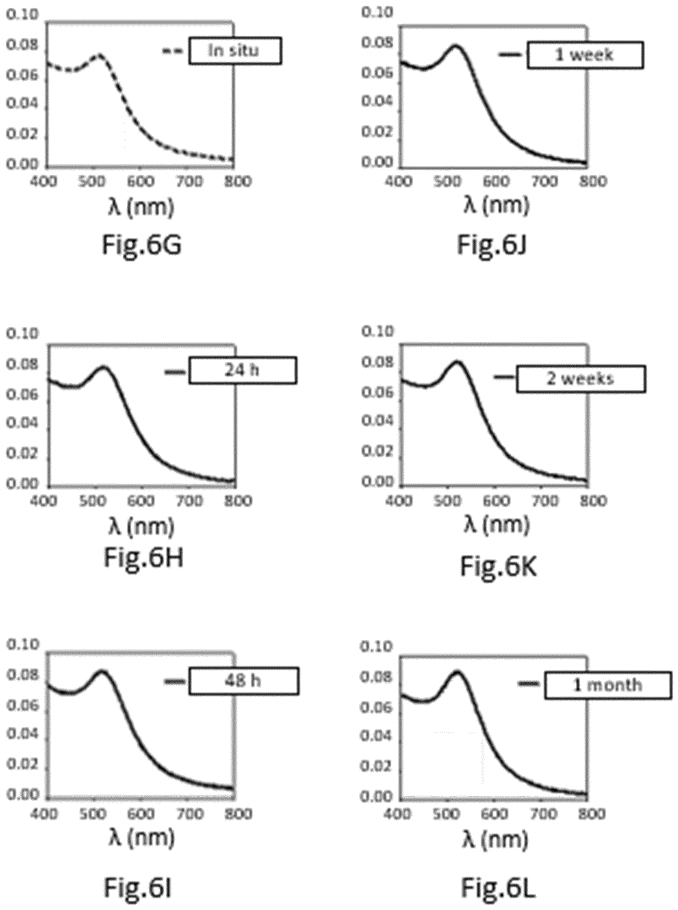

Figure 6G shows the formation and stability of the nanosystem Au@16-3-16/miR-21, R = 0.378, *in situ.*
Figure 6H shows the formation and stability of the nanosystem Au@16-3-16/miR-21, R = 0.378, at 24 hours.
Figure 6l shows the formation and stability of the nanosystem Au@16-3-16/miR-21, R = 0.378, at 48 hours.
Figure 6J shows the formation and stability of the nanosystem Au@16-3-16/miR-21, R = 0.378, at 1 week.
Figure 6K shows the formation and stability of the nanosystem Au@16-3-16/miR-21, R = 0.378, at 2 weeks.
Figure 6L shows the formation and stability of the nanosystem Au@16-3-16/miR-21, R = 0.378, at 1 month.

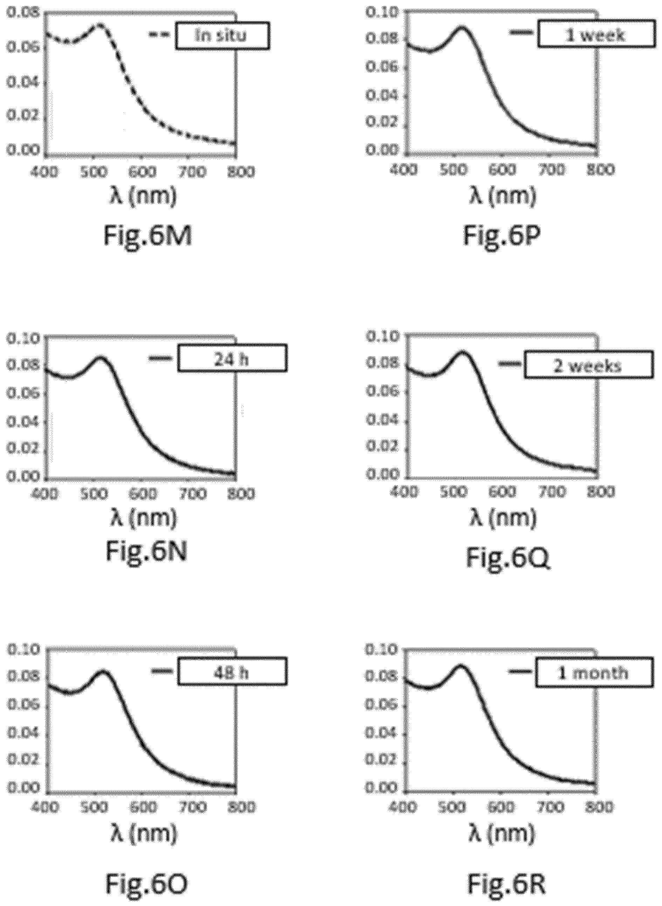

Figure 6M shows the formation and stability of the nanosystem Au@16-3-16/miR-21, R = 0.283, *in situ.*
Figure 6N shows the formation and stability of the nanosystem Au@16-3-16/miR-21, R = 0.283, at 24 hours.
Figure 6O shows the formation and stability of the nanosystem Au@16-3-16/miR-21, R = 0.283, at 48 hours.
Figure 6P shows the formation and stability of the nanosystem Au@16-3-16/miR-21, R = 0.283, at 1 week.
Figure 6Q shows the formation and stability of the nanosystem Au@16-3-16/miR-21, R = 0.283, at 2 weeks.
Figure 6R shows the formation and stability of the nanosystem Au@16-3-16/miR-21, R = 0.283, at 1 month.

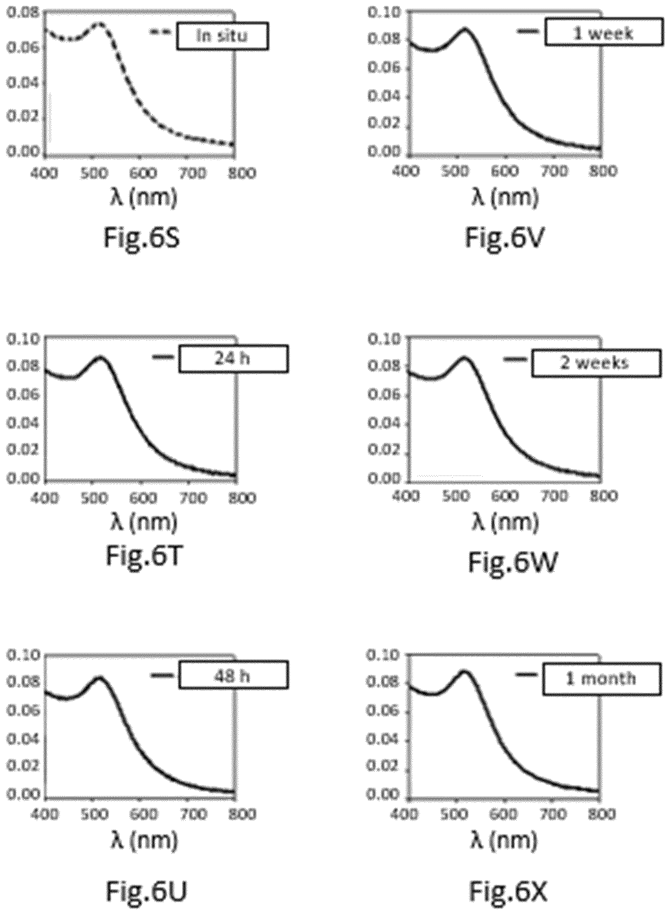

Figure 6S shows the formation and stability of the nanosystem Au@16-3-16/miR-21, R = 0.189, *in situ.*
Figure 6T shows the formation and stability of the nanosystem Au@16-3-16/miR-21, R = 0.189, at 24 hours.
Figure 6U shows the formation and stability of the nanosystem Au@16-3-16/miR-21, R = 0.189, at 48 hours.
Figure 6V shows the formation and stability of the nanosystem Au@16-3-16/miR-21, R = 0.189, at 1 week.
Figure 6W shows the formation and stability of the nanosystem Au@16-3-16/miR-21, R = 0.189, at 2 weeks.
Figure 6X shows the formation and stability of the nanosystem Au@16-3-16/miR-21, R = 0.189, at 1 month.

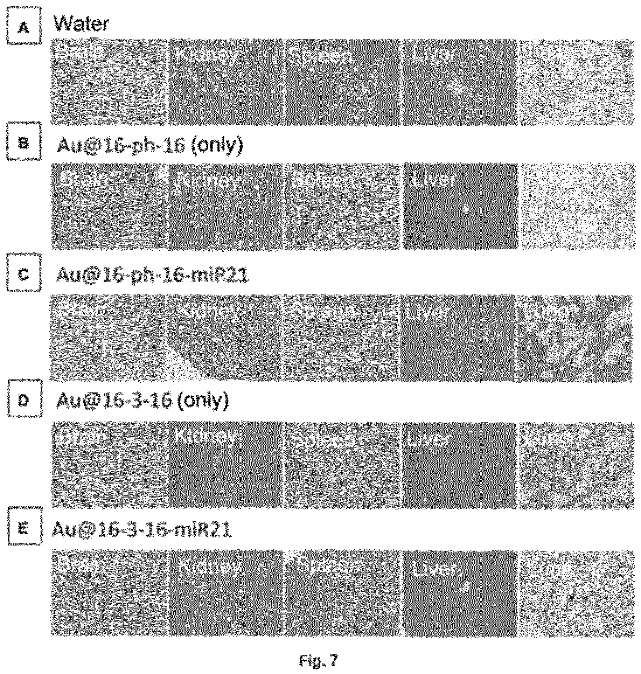

Figure 7 shows microscopic images representative of all liver, lung, brain, spleen and kidney tissues; corresponding to hematoxylin and eosin staining. (A) Water, (B) Au@16-ph-16 (only), (C) Au@16-ph-16 miR21, (D) Au@16-3-16 (only), (E) Au@16-3-16 miR21.

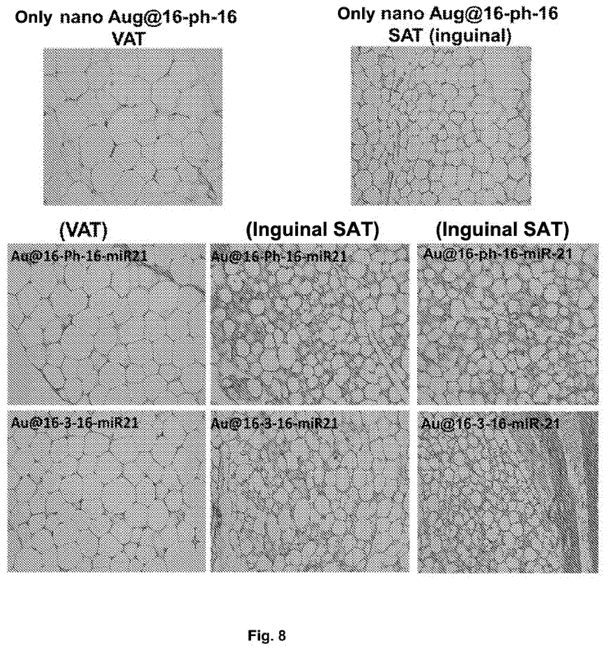

Figure 8 shows microscopic images representative of the histological sections of visceral white fats (VAT) and subcutaneous white fats (Inguinal). Hematoxylin and eosin staining.

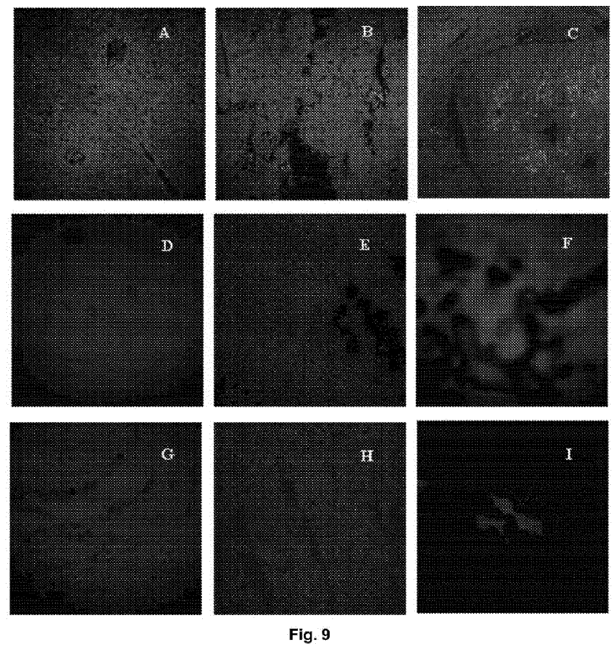

Figure 9 shows the CARS (Coherent Anti-Stokes Raman Scattering) images of different tissues of mice after 48 hours of treatment with Au@Gemini/miR-21 (R = 0.30) and untreated control tissues. (A) PBS in control tissue; (B) Au@16-3-16/miR-21 in control tissue; (C) Au@16-Ph-16/miR-21 in control tissue; (D, G) Livers obtained from (D) Au@16-3-16/miR-21 and (G) Au@16-Ph-16/miR-21 mice; (E, H) Spleens obtained from mice treated with (E) Au@16-3-16/miR-21 and (H) Au@16-Ph-16/miR and (F, I) SAT fat obtained from (F) Au@16-3-16/miR-21 and (I) mice treated with Au@16-Ph-16/miR.

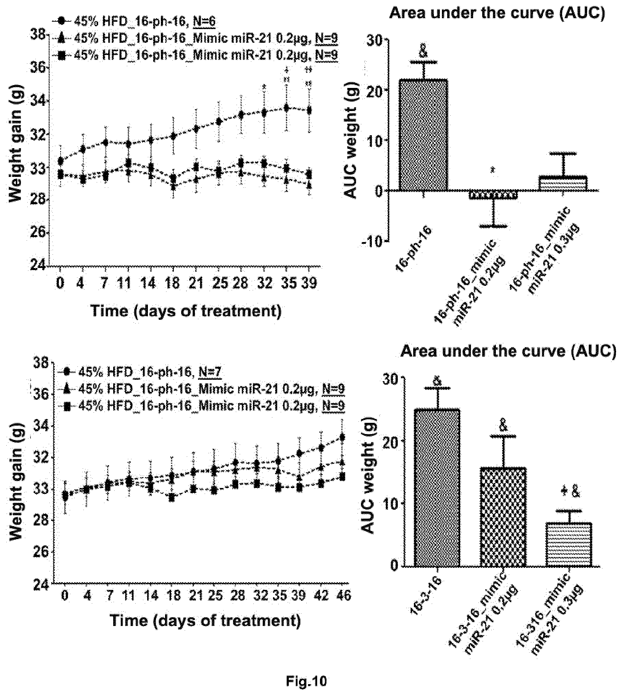

Figure 10 shows *<0.05 **<0.01 45% HFD Au@16-ph-16 with respect to 45% HFD miR-21 mimetic 0.2 ug +<0.05 ++<0.01 45% HFD Au@16-ph-16 with respect to 45% HFD miR-21 mimetic 0.3 ug and it changes in comparison with the baseline value (0), i.e., the mice in the control group show a significant weight gain with HFD in comparison with the baseline value 0 which is the initial weight of each mouse.

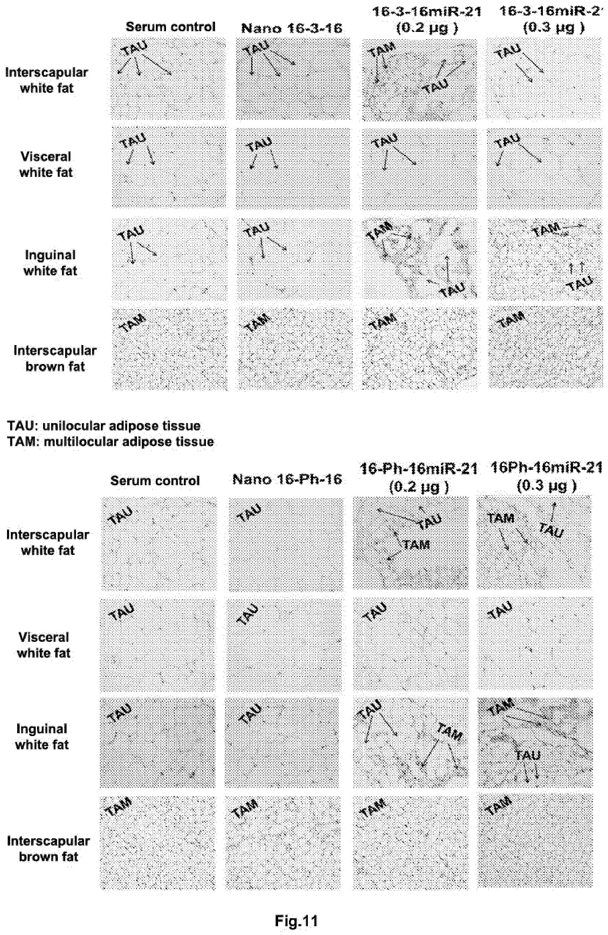

Figure 11 shows microscopic images representative of the histological sections of interscapular white fat, visceral white fat, inguinal subcutaneous white fat and interscapular brown fat; with hematoxylin and eosin staining.

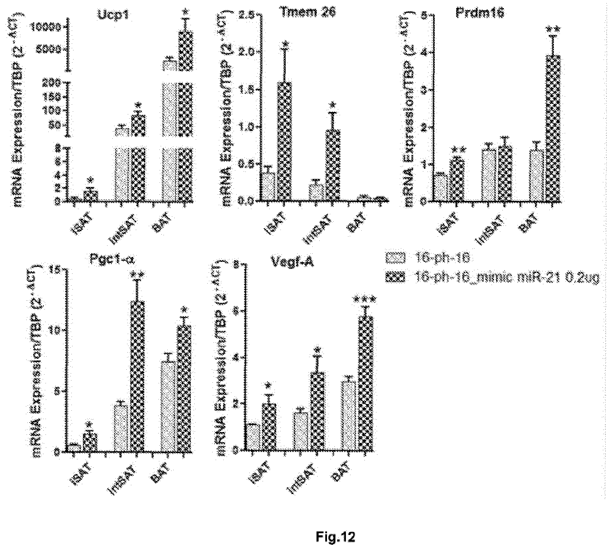

Figure 12 shows gene expression analysis with messenger RNA extracted from inguinal subcutaneous adipose tissues (ISAT) and interscapular subcutaneous adipose tissues (int. SAT), as well as from interscapular brown adipose tissue of mice treated *in vivo* with the nanosystem Au@16-Ph-16-miR-21 and a control with Au@16-Ph-16 without miRNA. The gene expression of Ucp1, Tmem26, Prdm16, Pgc1-a and Vegf-A genes has been quantified by means of real-time PCR.

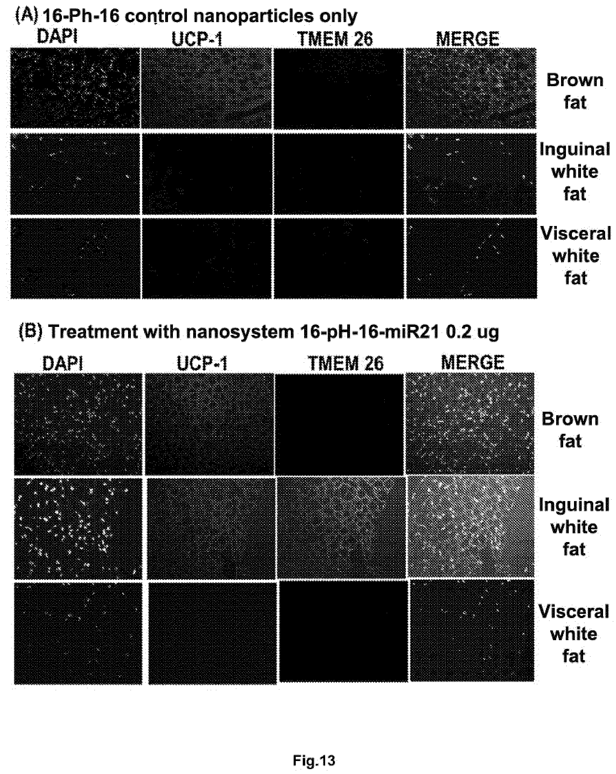

Figure 13 shows protein expression analysis by means of immunohistochemical images of brown adipose tissue, inguinal subcutaneous white adipose tissue and visceral white adipose tissue of mice treated *in vivo* with the nanosystem Au@16-Ph-16-miR-21 and a control with Au@16-Ph-16 without miRNA. Antibodies specific for the UCP-1 protein (labelled in red), for the TMEM26 protein (labelled in green), for the DNA content with 4',6-diamino-2-phenylindole or DAPI (labelled in blue) and a labelling containing the three preceding ones, called MERGE, were used.

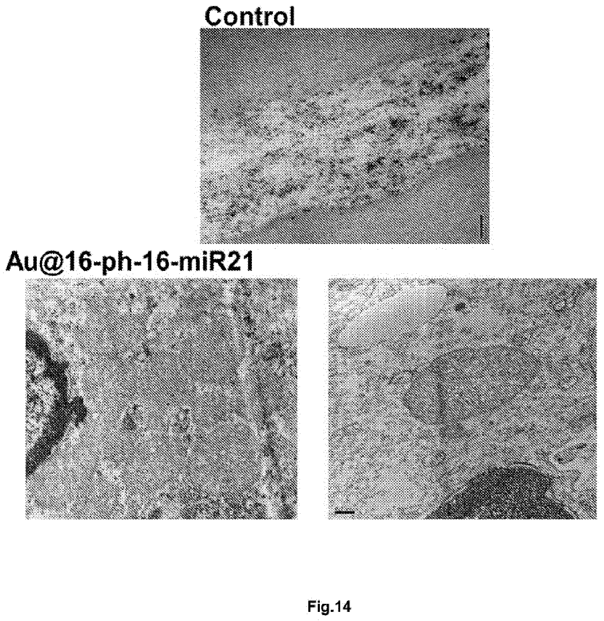

Figure 14 shows electron micrograph images of the abundance of mitochondria in subcutaneous white fat (inguinal) of control mice and in mice treated with the nanosystem Au@16-pH-16-miR-21.

### DETAILED DESCRIPTION OF EMBODIMENTS/EXAMPLES

### Example 1. Characterisation and verification of the stability of the nanosystem

The process for synthesising the nanosystems begins with the prior synthesis of Gemini surfactants and uses HAuCl₄ to provide the gold content of the nanoparticle. First, 390 µl of an aqueous HAuCl₄ solution with a concentration of 23 mM prepared in an aqueous solution were taken, to which there were added 30 ml of the Gemini surfactant 16-Ph-16 or 16-3-16 at the concentration of 4·10⁻⁵ M and 4·10⁻⁴ M, respectively. Said preparation was subjected to vigorous continuous stirring for 5 minutes in the absence of light, a clear bright yellow solution being obtained as a result. Next, 100 µl of an aqueous sodium borohydride solution 0.4 M are added dropwise, keeping the mixture under moderate stirring for 15 minutes in the dark. During this time interval, the mixture turns from yellow to a clear reddish colour. As a result, precursor gold nanoparticles called Au@16-Ph-16 and Au@16-3-16 were obtained, in this example at concentrations of 5.6·10⁻⁸ M and 1.7·10⁻⁷ M, respectively.

Starting from the synthesis of the precursor nanosystems, nanosystems functionalised with miRNA biomolecule in different proportions were prepared. Complexing with miR-21 was performed by working in polymer excess conditions at all times. Therefore, starting from a fixed amount of precursor nanosystem, C_{AU@m-sm} = 5.6·10⁻⁹ M and 1.7·10⁻⁸ M for Au@16-Ph-16 and Au@16-3-16, respectively, variable amounts of the biopolymer, C_{miR-21} = 4.5·10⁻⁸, 6.0·10⁻⁸ and 9.0·10⁻⁸ were added. As a result, the following preparations corresponding to the following proportions in analytical concentration ratio, R = C_{AU@m-s-m} /C_{miR-21} for each system, were obtained: R = 0.124, 0.093 and 0.063 for Au@16-Ph-16/miR-21; and R = 0.378, 0.283 and 0.189 for Au@16-3-16/miR-21. Stable Au@m-s-m/miR-21 type complexes were thereby obtained by continuous stirring, where a total of 30 minutes of gentle stirring at room temperature was required for obtaining a preparation volume of 30 ml. Next, 24 hours lapse before injection into test individuals, with the prior conditioning of the nanosystem at a temperature of 4°C being fundamental. A moderate change to a slightly purplish tone is indicative of the stabilisation and formation of the resulting nanosystem functionalised with miR-21.

The stability of gold nanoparticles coated with a surfactant (16-Ph-16@AuNPs and 16-3-16@AuNPs), as well as nanoparticles coated with a Gemini surfactant and miR-21 (miR-21/16-Ph-16@AuNPs and miR-21/16-3-16@AuNPs) was evaluated by means of UV-visible spectroscopy, following the shape and wavelength of the maximum of the surface plasmon band over time.

The absorbance spectra were prepared using a CARY 500 SCAN UV-vis-NIR spectrophotometer (Varian). Data was collected every 2 nm with a standard 10 mm thick glass cell and the spectra were recorded in the wavelength range of 800 to 400 nm. The surface plasmon resonance exhibited by the nanosystems was shown as a strong absorption band in the visible region. Wavelength precision and spectral bandwidth were ± 0.3 nm and 0.5 nm, respectively.

The absorption spectra of each sample were measured for 30 minutes, in the worst cases, and the position of the maximum surface plasmon was verified for at least one month in sterility conditions, with no noticeable changes being observed in the original position of the surface plasmon band or in the wavelength of the maximum absorption. Therefore, no evidence of aggregation was observed in any of the studied nanosystems.

The experiments were carried out in an aqueous solution at a fixed colloidal gold concentration of 5.6·10⁻⁹ M and 5.6·10⁻⁸ M for systems 16-Ph-16 and 1.7·10⁻⁸ M and 1.7·10⁻⁷ M for the corresponding gold nanoparticle systems 16-3-16, respectively. Furthermore, the spectra of the miR-21/16-Ph-16-AuNP and miR-21/16-3-16-AuNP complexes were prepared with different molar ratios (defined as R = C_{Au-Gemini}/C_{miR-21}): R = 0.124, 0.093 and 0.063 for miR-21-16-Ph-16-AuNPs and R = 0.378, 0.283 and 0.189 for miR-21-16-3-16-AuNPs.

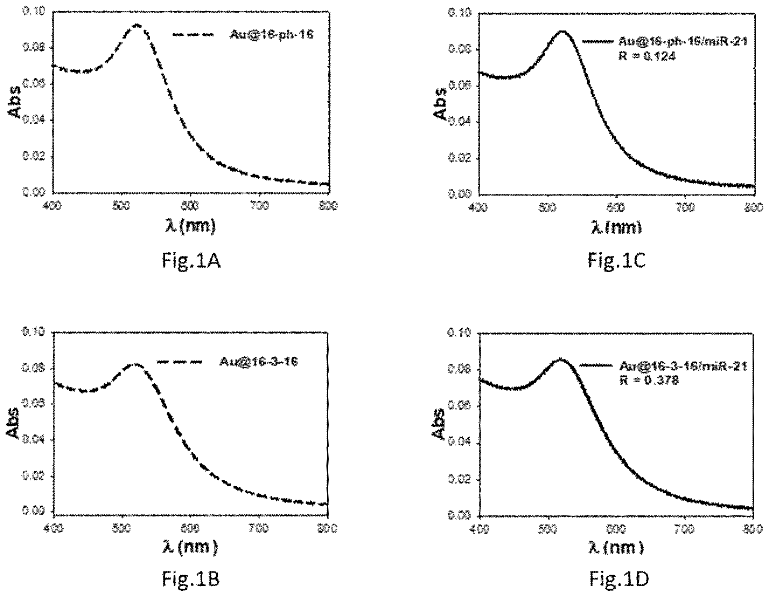

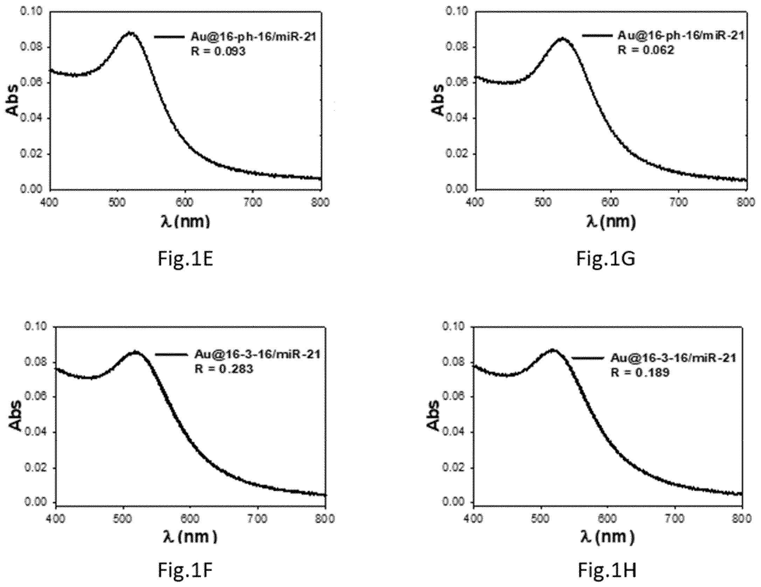

The UV-visible graphs shown in Figures 1A-1H show that the position of the plasmon peak (λspr) of the nanosystems coated with Gemini surfactants (Au@16-3-16 and Au@16-Ph-16) is similar to a value of about 519 nm. According to the H. Wolfgang correlation, λspr = 512 + 6.53·exp (0.0216xd), where d is the diameter of the gold core, a λspr = 519 nm corresponds to a AuNP having a core size of about 3.2 nm. It is of interest to point out that in the different Au@16-3-16/miR-21 preparations, no modification whatsoever was observed in the position of the maximum λspr, but an increase in absorbance intensities in reference to the corresponding Au@16-3-16 precursor was observed, which indicates the relative formation of Au@16-3-16-miR-21 complexes (see Figure 1A). However, in the case of Au@16-Ph-16 nanosystems, a shift of about 2 nm in the position of maximum absorption is observed (λspr = 521 nm) for preparations corresponding to R = 0.124-0.093 and of 10 nm (λspr = 529 nm) for R = 0.063, all this together with a reduction in absorbance intensity since these nanosystems are modified with miR-21 at different proportions thereof (see Figure 1B). The increase observed in the positions of maximum wavelength without significant modification in the SPR band demonstrates that the Gemini surfactant/miR-21 complexes are correctly formed and no significant aggregation processes have been produced.

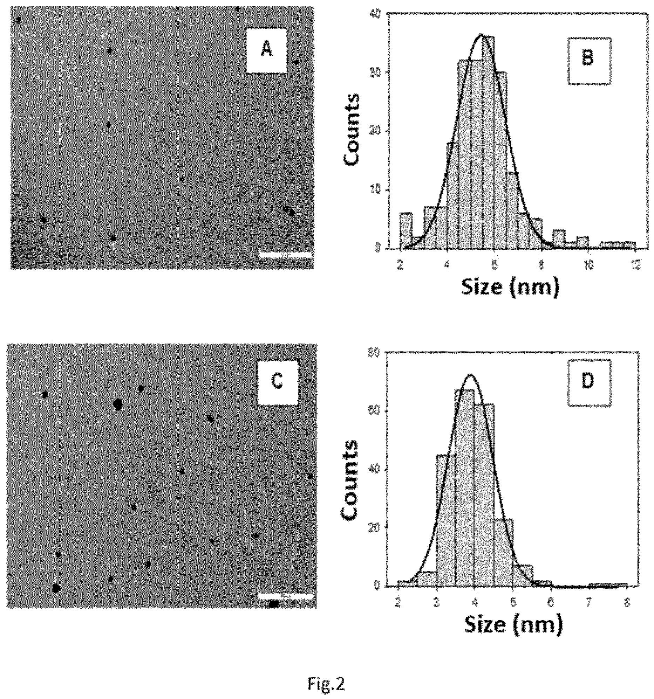

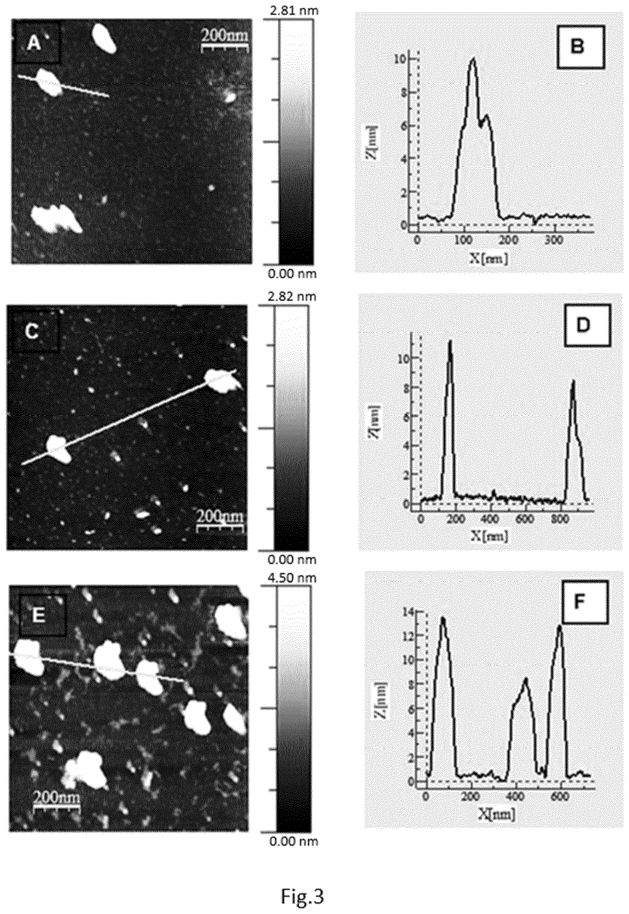

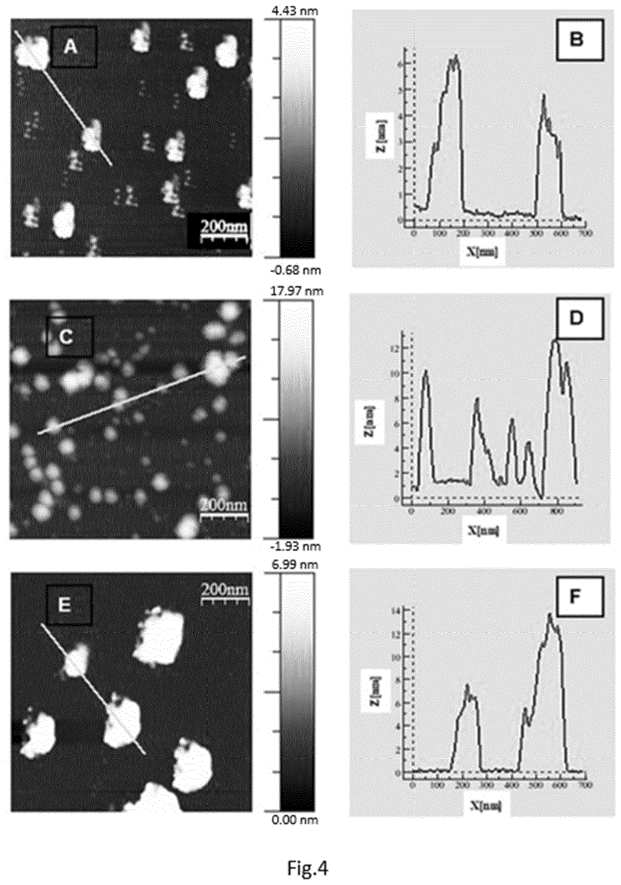

Moreover, the different nanosystems were characterised to determine both the morphology and the size and charge of the resulting complex using different structural techniques and in solution. Specifically, TEM and AFM microscopy techniques were used for determining the size and shape of the complexes. It was therefore determined that nanoparticles Au@16-3-16 and Au@16-Ph-16 have an average size of 3.8 ± 0.5 nm and 5.5 ± 0.5 nm, respectively (Figure 2). Moreover, the formation of the Au@16-3-16/miRNA and Au@16-Ph-16/miRNA complex (Figures 3 and 4) is proven. The transverse section analysis shows nanosystems having a thickness which matches the diameter of the nanoparticles obtained by means of TEM, while at the same time showing miRNA binding, with Au@16-3-16/miRNA and Au@16-Ph-16/miRNA complexes being obtained, the average sizes of which in the x-y direction are about 50 nm and 60 nm, respectively. The results obtained by means of the technique of scattering light in solution, DLS, confirms the results obtained by microscopy.

For TEM examination, a droplet (10 µl) of the aqueous gold nanoparticle solution was placed on a copper grid coated with a carbon film, which was then left to air dry for a few hours at room temperature. TEM analysis was carried out in a Philips CM electron microscope working at 200 kV, and the resulting images were analysed using the free ImageJ software.

AFM images were obtained with Molecular Imaging Picoscan 2500 (Agilent Technologies). Silicon cantilevers (model Pointprobe, Nanoworld) with a resonance frequency of about 240 kHz and a nominal force constant of 42 N/m were used. All AFM images were taken in the air and in the tapping mode, with a scan speed of about 0.5 Hz and data collection speed at 256 × 256 pixels.

Lastly, the dynamic light scattering (DLS) technique and Zeta potential measurements were used to evaluate the size distribution and charge of the precursor nanosystems and nanosystems functionalised with miR-21. For DLS measurements, Zetasizer Model ZS-90 (Malvern Instrument, Ltd., United Kingdom) equipment was used. The sample was illuminated with a laser with a fixed detection arrangement of 90° towards the centre of the area of the cell to analyse fluctuation in scattered light intensity. At least 5 size measurements were taken for each sample, and the relative error for the hydrodynamic diameter was calculated to be < 5%. The results were obtained in terms of average hydrodynamic diameters, with the percentage of the different complexes obtained in solution being obtained. DTS1060 capillary polycarbonate cell was used, and the samples were introduced in molar ratios identical to the UV-visible tests.

The zeta potentials of the different samples reveal the formation of highly positively charged structures (Table 1), which allows these nanosystems to be potential vectors for transporting medicinal products to the cell.

**Table 1. DLS size distribution and Zeta potential per number of free Au@Gemini nanoparticles and nanoparticles functionalised with miR-21 in different molar ratios in water (defined as R = C_{Au@Gemini}/C_{miR-21}).**

| | Au@Germini/miR-21 nanosystem | | |
|---|---|---|---|
| | CmiR-21 = 4.5·10⁻⁸ M | CmiR-21 = 6.0·10⁻⁸ M | C_{miR-21} = 9.0.10⁻⁸ M |
| Au@16-3-16 type nanosystems | R = 0.378 | R = 0.283 | R = 0.189 |
| | (46 ± 5) mV (18 ± 3) nm; 8% | (35.6 ± 1.1) mV | (50.5 ± 1.7) mV |
| | (4.6 ± 1.4) nm 92% | (5.0 ± 0.7) nm | (11 ± 4) nm |
| Au@16-Ph-16 type nanosystems | R = 0.124 | R = 0.093 | R = 0.063 |
| | (43 ± 6) mV (22 ± 2) nm; 47% | (35 ± 4) mV | (31.4 ± 1.2) mV |
| | (6.5 ± 1.4) nm 53% | (10.9 ± 1.6) nm | (13.3 ± 1.4) nm |

### Example 2. Efficiency of the nanosystem for complexing miRNA

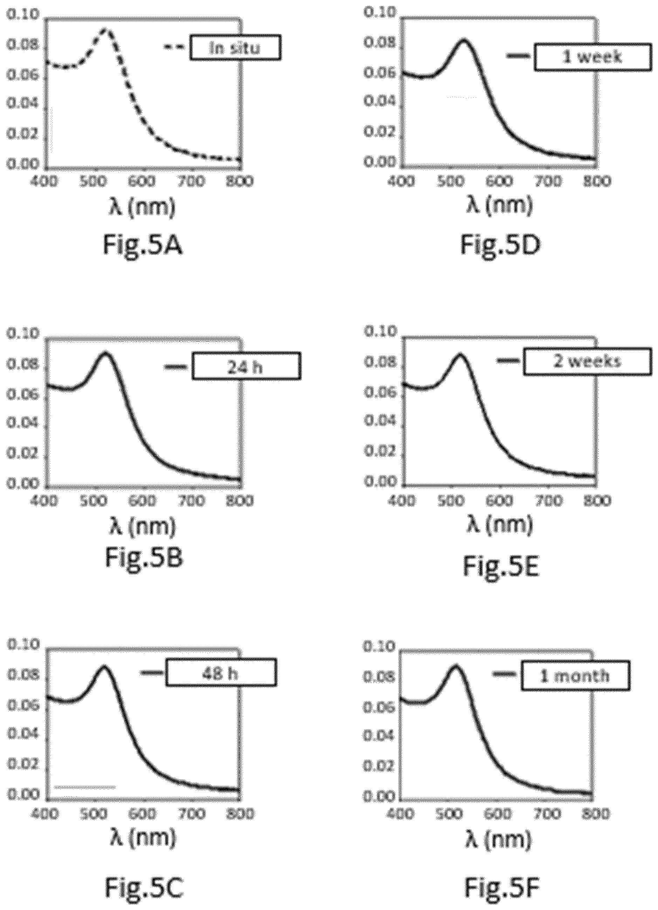

To verify the effective formation of different functionalised nanosystems UV-vis spectra were prepared as a function of time with respect to the nanosystems described in Example 1, and pertinent modifications in the surface plasmon band of the nanoparticle, as well as the stability thereof over time, were studied. Figures 5 and 6 show the tracking of the formation of the nanosystems functionalised with miR-21 from precursors Au@16-Ph-16 and Au@16-3-16, respectively. In the case of functionalised nanosystems having nanoparticle Au@16-Ph-16 as a precursor in different proportions of R (0.124, 0.093 and 0.063), the formation of the resulting nanosystem is clearly proven 24 hours after the end of the continuous stirring process (see Example 1), which is considered *"in situ"* or time zero preparation. In that sense, if the UV-vis spectra from the *in situ* mixture up to 24 hours of formation are compared, in the different preparations, a shift of the surface plasmon band towards blue, along with a clear reduction in band intensity (see Figures 5A-5X), are indicated. The observed hypochromic effect can be interpreted as a consequence of the neutralisation of phosphate groups of the miR-21 as it binds to the different positively and oppositely charged surfactants surrounding the precursor nanoparticle. The absence of significant changes in the position and shape of the band after 24 hours of mixing and up until at least one month of synthesis proves both the complete formation of the nanocomplex 24 hours after mixing and the stability thereof over time. On the other hand, if once stabilised, the position of the plasmon band of the precursor and functionalised nanosystems is compared, a bathochromic shift typical of intercalative processes is observed.

Similarly, the formation of functionalised nanosystems having as a precursor nanoparticle Au@16-3-16 in different proportions of R (0.378, 0.283 and 0.189) is also obvious 24 hours after the mixing process. In this case, the comparison of the spectra from *in situ* mixing up until t =24 hours shows an increase in surface plasmon band intensity, without any shift in the position of the band being shown (see Figures 6A-6X). This difference may be due to a change in the manner in which the surfactant binds to miR-21 which gives rise nanosystems having a more regular and dispersed structure, producing a hyperchromic effect in the absorption intensity of the plasmon band of the precursor Au@16-3-16.

### Example 3. Toxic effect of treatment using miRNA

**Table 2a: Short-term effect of miR-21-Nano on the weight of mice. Mice were treated daily for 7 days by means of subcutaneous injections of 200 µl of miR-21-Nano and were weighed after 48 h.**

| Code | Type of nanoparticles | Amount of MiR-21 (µg) | Weight before treatment (t = 0) | Weight after treatment (t = 48 h) |
|---|---|---|---|---|
| A1 | H₂O DEPC | 0 | 28.9 | 27.5 |
| A2 | Au@16-pH-16 | 0 | 26.1 | 25.5 |
| A3 | Au@16-pH-16 | 0.495 | 25.1 | 25.4 |
| A4 | Au@16-pH-16 | 0.22 | 25.5 | 25.8 |
| A5 | Au@16-pH-16 | 0.11 | 24.3 | 24.4 |
| A6 | Au@16-3-16 | 0 | 29.9 | 29.2 |
| A7 | Au@16-3-16 | 0.495 | 26.6 | 26.3 |
| A8 | Au@16-3-16 | 0.22 | 30.4 | 28.8 |
| A9 | Au@16-3-16 | 0.11 | 27.3 | 26.6 |
| S1 | Physiological saline solution | 0 | 26.7 | 25.6 |

| | | | | |
|---|---|---|---|---|
| DEPC: Diethyl pyrocarbonate | | | | |

Tests were performed to rule out the possible toxic effect of the nanosystem (Au@Gemini/miR-21 nanoparticles). C57BL/6J mice were injected daily for 7 days with nanosystem miR-21 having different miR-21 concentrations. The injections were subcutaneous injections in the interscapular part of the mouse. One group of mice was then sacrificed after 48 hours to evaluate the acute effect of said complex (Table 2a), and another group was sacrificed after one month to evaluate the chronic effect (Table 2b). In addition to control groups with serum and water, another group of mice treated with miR-21 conjugated with *in vivo*-JetPEI^{®} delivery reagent with an miR-21 concentration of 0.5 µg was included in the study.

**Table 2b: Long-term effect of miR-21-Nano on the weight of mice. Mice were treated daily for 7 days by means of subcutaneous injections of 200 µL of miR-21-Nano and weighed after 15 days and 30 days.**

| Code | Type of nanoparticles | Amount of miR-21 (µg) | Weight before treatment (t = 0) | Weight after treatment (t = 15 days) | Weight after treatment (t = 30 days) |
|---|---|---|---|---|---|
| B1 | H₂O DEPC | 0 | 25.7 | 26.7 | 27.2 |
| B2 | Au@16-pH-16 | 0 | 24.9 | 25.4 | 26 |
| B3 | Au@16-pH-16 | 0.495 | 26.4 | 27.2 | 27.3 |
| B4 | Au@16-pH-16 | 0.22 | 28 | 28.8 | 28.6 |
| B5 | Au@16-pH-16 | 0.11 | 26.7 | 27.5 | 27.1 |
| B6 | Au@16-3-16 | 0 | 27 | 28.7 | 28.6 |
| B7 | Au@16-3-16 | 0.495 | 29.2 | 29.7 | 30.5 |
| B8 | Au@16-3-16 | 0.22 | 27 | 28.3 | 28.6 |
| B9 | Au@16-3-16 | 0.11 | 24 | 26.9 | 27.3 |
| S2 | Physiological saline solution | 0 | 26.8 | 28.3 | 27.9 |
| 1 | jetPei | 0.5 | 24.1 | 27 | 27.5 |
| 2 | jetPei | 0.5 | 25.9 | 28.2 | 27.4 |
| 3 | jetPei | 0.5 | 26.2 | 27.7 | 27.9 |
| 4 | jetPei | 0.5 | 26 | 27.2 | 27.9 |
| 5 | jetPei | 0.5 | 25 | 25 | 27.2 |

With these tests, no alterations were observed in the normal coat, behaviour, activity, weight level of the mice, no sign of depression and no palpable node was detected. Furthermore, no mouse died before the end of the study.

Blood samples were analysed before the mice were sacrificed in order to perform the corresponding blood counts, and the values were compared with those of the reference group (Table 3), without any changes being observed.

**Table 3a: Short-term effect of miR-21-Nano on the blood composition of mice. Blood count corresponding to mice treated daily for 7 days by means of subcutaneous injections of 200 µl of miR-21-Nano and after 48 h.**

| Type of nanoparticles | H₂O d.p.c. | Physiological saline solution | Au@ 16-ph-16 | Au@ 16-3-16 | Au@ 16-ph-16-miR-21-0.5 | Au@ 16-ph-16-miR-21-0.2 | Au@ 16-ph-16-miR-21-0.1 | Au@ 16-3-16-miR-21-0.5 | Au@ 16-3-16-miR-21-0.2 | Au@ 16-3-16-miR-21-0.1 | REF. VAL-UES (n=4) |
|---|---|---|---|---|---|---|---|---|---|---|---|
| Code | A1 | S1 | A2 | A6 | A3 | A4 | A5 | A7 | A8 | A9 | |
| RBC (×10⁶/µl) | 9.21 | 9.33 | 10.06 | 9.08 | 10.03 | 8.98 | 10.01 | 8.69 | 8.14 | 10.05 | 9.84 ± 0.35 |
| HEMAT-OCRIT (%) | 42.7 | 42.7 | 45.2 | 41.3 | 45.5 | 40.2 | 45.2 | 38.9 | 36.4 | 45.4 | 45.95 ± 1.34 |
| HEMOGLOBIN (g/dl) | 14.2 | 14.1 | 15.5 | 13.5 | 15.3 | 13 | 15 | 12.7 | 11.2 | 15.4 | 14.37 ± 0.67 |
| MCV (fl) | 46.4 | 45.5 | 44.9 | 45.5 | 45.4 | 44.8 | 45.2 | 44.8 | 44.7 | 45.2 | 46.75 ± 0.40 |
| MCHC (d/dl) | 33.2 | 33.2 | 34.3 | 32.6 | 33.6 | 32.3 | 32.6 | 32.6 | 30.8 | 33.9 | 31.2 ± 0.59 |
| MCH (pg) | 15.4 | 15.1 | 15.4 | 14.8 | 15.3 | 14.4 | 15 | 14.6 | 13.8 | 15.3 | 14.57 ± 0.23 |
| RDW (%) | 14.4 | 12.7 | 12.9 | 13.7 | 11.8 | 13.9 | 12.8 | 13.9 | 14 | 13.8 | 13.82 ± 0.36 |
| WBC (×10³/µl) | 1.62 | 2 | 1.98 | 1.75 | 1.13 | 2.64 | 1.48 | 4.4 | 1.88 | 3.54 | 3.54 ± 0.24 |
| NEUTRO-PHILS (%) | 9.56 | 37.12 | 9.79 | 10.1 | 19.68 | 6.98 | 11.86 | 10.1 | 7.08 | 5.03 | 17.46 ± 2.98 |
| EOSIN-OPHILS (%) | 0 | 0 | 0.49 | 0.17 | 1.71 | 0.72 | 1.6 | 0.31 | 0.93 | 0.89 | 0.71 ± 0.16 |
| BASO-PHILS (%) | 0 | 0 | 0 | 0.46 | 0 | 0 | 0 | 0.52 | 0 | 0 | 0.29 ± 0.10 |
| LYMPHOCYTES (%) | 89.31 | 62.44 | 88.12 | 88.88 | 76.38 | 90.04 | 85 | 84.44 | 90.42 | 89.7 | 78.58 ± 3.38 |
| MONOCYTES (%) | 1.13 | 0.44 | 1.6 | 1.64 | 2.61 | 1.9 | 1.5 | 1.45 | 1.57 | 4.38 | 2.96 ± 0.89 |
| PLATELETS | 198 | 147 | 468 | 84 | 105 | 160 | 144 | 131 | 152 | 298 | 187.75 ± 32.49 |
| PLATELET-CRIT (%) | 0.15 | 0.11 | 0.39 | 0.06 | 0.06 | 0.11 | 0.11 | 0.08 | 0.12 | 0.21 | 0.15 ± 0.03 |
| MPV (fl) | 7.6 | 7.5 | 8.4 | 7.2 | 5.9 | 7 | 7.7 | 6.6 | 7.6 | 7.1 | 8.32 ± 0.12 |

**Table 3b: Long-term effect of miR-21-Nano on the blood composition of mice. Blood count corresponding to mice treated daily for 7 days by means of subcutaneous injections of 200 µl of h miR-21-Nano and after 30 days.**

| Type of nanoparticles | H₂O d.p.c. | Physiological saline solution | Au@ 16-ph-16 | Au@ 16-3-16 | Au@ 16-ph-16-miR-21-0.5 | Au@ 16-ph-16-miR-21-0.2 | Au@ 16-ph-16-miR-21-0.1 | Au@ 16-3-16-miR-21-0.5 | Au@ 16-3-16-miR-21-0.2 | Au@ 16-3-16-miR-21-0.1 | REF. VAL-UES (n=4) |
|---|---|---|---|---|---|---|---|---|---|---|---|
| Code | B1 | S2 | B2 | B6 | B3 | B4 | B5 | B7 | B8 | B9 | |
| RBC (×10⁶/µl) | 9.27 | 8.87 | 9.28 | 10 | 9.77 | 9.97 | 8.8 | 10.52 | 10.14 | 8.76 | 9.84 ± 0.35 |
| HEMAT-OCRIT (%) | 45.8 | 39.6 | 44.3 | 46.2 | 45 | 46.9 | 41.6 | 48.2 | 46.8 | 44.6 | 45.95 ± 1.34 |
| HEMOGLOBIN (g/dl) | 14.6 | 13.6 | 15.4 | 15.2 | 14.6 | 15.2 | 14.6 | 15.3 | 15.3 | 14.9 | 14.37 ± 0.67 |
| MCV (fl) | 49.5 | 44.7 | 47.8 | 46.2 | 46.1 | 47.1 | 47.3 | 45.9 | 46.2 | 51 | 46.75 ± 0.40 |
| MCHC (d/dl) | 31.8 | 34.3 | 34.7 | 32.9 | 32.4 | 32.4 | 35 | 31.7 | 32.6 | 33.4 | 31.2 ± 0.59 |
| MCH (pg) | 15.7 | 15.3 | 16.5 | 15.2 | 14.9 | 15.2 | 16.5 | 14.5 | 15 | 17 | 14.57 ± 0.23 |
| RDW | 12.6 | 14 | 14.9 | 12.5 | 13.5 | 12.3 | 13.2 | 12.6 | 12.6 | 13.1 | 13.82 ± |
| (%) | | | | | | | | | | | 0.36 |
| WBC | 2.63 | 3.12 | 3.07 | 2.61 | 1.83 | 4.78 | 3.13 | 3.76 | 3.29 | 2.61 | 3.54 ± 0.24 |
| NEUTRO-PHILS (%) | 21.5 | 29.82 | 25.2 | 17.66 | 14.4 | 14.24 | 24.15 | 21.46 | 13.49 | 21.2 | 17.46 ± 2.98 |
| EOSIN-OPHILS (%) | 0.91 | 0.64 | 2.05 | 0.69 | 14.65 | 1.23 | 0.97 | 2.99 | 4.24 | 4.18 | 0.71 ± 0.16 |
| BASO-PHILS (%) | 0.27 | 0.64 | 0.38 | 0.26 | 0.23 | 0.41 | 0.41 | 0.12 | 0.12 | 0.11 | 0.29 ± 0.10 |
| LYMPHOCYTES (%) | 63.66 | 58.19 | 77.81 | 72.57 | 66.47 | 74.29 | 71.68 | 63.09 | 70.26 | 67.46 | 78.58 ± 3.38 |
| MONOCYTES (%) | 13.66 | 10.71 | 2.56 | 8.82 | 4.25 | 9.83 | 2.79 | 12.34 | 11.89 | 7.05 | 2.96 ± 0.89 |
| PLATELETS | 162 | 857 | 812 | 384 | 191 | 792 | 618 | 667 | 827 | 351 | 187.75 ± 32.49 |
| PLATELET-CRIT (%) | 0.11 | 0.71 | 0.67 | 0.32 | 0.15 | 0.67 | 0.55 | 0.53 | 07 | 0.28 | 0.15 ± 0.03 |
| MPV (fl) | 7.3 | 8.3 | 8.3 | 8.5 | 8 | 8.5 | 8.9 | 8 | 8.5 | 8 | 8.32 ± 0.12 |

Morphological studies consisting of performing the hematoxylin and eosin histochemical study (Figures 7A-D) to assess possible anomalies at the tissue level were then performed. This allowed verifying, by means of optical microscopy, whether the different tissues present structural or morphological anomalies susceptible to pathology. To that end, several organs will be extirpated: liver, lung, brain, spleen, kidney, visceral fat and inguinal fat. The organs have not experienced any alteration during treatment, with the most significant modifications being observed at the inguinal adipose tissue level, in which the appearance of multilocular adipose tissue (corresponding to beige adipose tissue) with 7 days of treatment with nanosystems Au@16-Ph-16-miR-21 and Au@16-3-16-miR-21 stands out (Figure 8).

To detect the possible accumulation of nanoparticles in tissues, Raman spectrometry in 10-micron tissue slices will be used. CARS (Coherent Antistokes Raman Spectroscopy) microscopy allowed obtaining high-resolution images in freshly extirpated tissues without the need for labelling or prior preparation with a chromophore, in turn allowing the spatial distribution of the specific component selected for mapping the sample in the selected image area to be detected (Figure 9).

Liver and lung tissues freshly extirpated from mice were dissected into thin tissue slices of about 10 pm thick and then submerged in PBS and placed on a glass slide for examination. To carry out the technique, there two parallel lines of independent laser excitation (Ti: Sapphire laser, model Tsunami, Spectra Physics) will be used, generating pulses of between 3 and 13 picoseconds that must be very carefully overlapped in time and space, i.e., they must precisely strike the same point of the sample and furthermore pulsate simultaneously, so that they generate the suitable CARS signal upon striking the sample. The beams are optically coupled to a vertical microscope (Olympus, model BX61WI) with ultrafast galvanometric mirror scanning. By setting the pump energy at 727nm (with a pulse width of about 4.5 ps and a microscope inlet power of 60 mW) and the energy of the stokes has been selected at 858 nm (with a pulse width of about 8 ps and a microscope inlet power 80 mW) for observing vibration of 2100 cm⁻¹ (CARS signal at 631 nm), for detecting gold nanoparticles in tissues. It could be confirmed through these tests that the nanoparticles did not accumulate in tissues.

*In vitro* analyses and accumulation at the tissue level in animal models are fundamental in understanding the toxicity level of the nanosystems. As is known, gold nanoparticles improve the Raman anti-Stoke signal of neighbouring amino acids, so the application of a suitable control CARS technique can detect the presence of AuNPs which measure this effect. To verify the possible accumulation of functionalised gold nanoparticles in different organs of mice, *ex vivo* CARS microscopy experiments were performed in different freshly dissected tissues after 48 hours of treatment (see Figure 9). Furthermore, some control experiments were carried out *in situ* by directly depositing gold nanoparticles and PBS buffer control tissues (see Figures 9A-C).

As expected, the CARS images of the PBS did not show a significant contrast in non-resonant condition (see Figure 9A), whereas the CARS signal clearly improved in the presence of Au@16-Ph-16-miR-21 and Au@16-3-16-miR-21 nanoparticles in control tissue (see Figures 9A-C), showing improved bright spots. The localised improvement of the anti-Stroke Raman signal at an excitation wavelength of 858 nm was observed for extirpated livers, spleens, lungs, brains, kidneys and VAT and SAT fatty tissues of the miR-21-AuNP derivatives (see Figures 9D-I) in the highest concentration of CmiR-21 (R 0.189 and 0.062 for Au@16-3-16-miR-21 and Au@16-Ph-16-miR-21, respectively) and treated mouse AuNP precursors. As a result, the Raman signal was completely absent for all the studied tissues treated with coated and non-coated miR-21 nanosystems after 48 hours of treatment. In this sense, the evidence of the present invention indicates that nanoparticles do not accumulate in the main organs under study, and this indicates the absence of toxicity due to the possible accumulation effect of the nanosystems of the present invention. However, more experiments must be conducted to evaluate a complete toxicity characterisation.

All in all, the CARS images of the "control" did not show a significant contrast in non-resonant condition, whereas the CARS signals of AuNPs increased considerably, appearing as bright spots scattered across the images taken of samples treated with AuNPs. The improvement probably resulted from the substantial scattering of AuNPs and the high third-order polarisability of AuNPs. In conclusion, the data demonstrated that these nanosystems did not show any short-term and long-term toxic effect on treated mice, and that the gold nanoparticles do not accumulate in tissues.

### Example 4. Study of the effect of in vivo treatment with nanosystems Au@16-Ph-16-miR-21 and Au@16-3-16-miR-21 on weight and fat gain in a mouse model of obesity subjected to a high fat diet

Male C57BL/6J mice obtained from Jackson Laboratory were used in the study. C57BL/6J is an animal model that is widely used in obesity and diabetes type 2 study. The animals were housed individually and kept in photoperiods of 12 hours of light and 12 hours of darkness at 24°C and 45% ± 5% humidity. During the 7 days of adaptation, the animals followed the normal diet used in the animal facility. The animals were then subjected to a high fat diet (HFD, 45% Kcal) for 53-55 days to generate the obese phenotype. The mice were weighed and treated 2 times a week for 6 weeks with 0.2 µg or 0.3 µg of miR-21 mimetic conjugated with nanosystems Au@16-Ph-16 or Au@16-3-16 in a final volume of 200 µl by means of subcutaneous injections (interscapular or inguinal). Adipose tissues (inguinal subcutaneous and interscapular, visceral and brown) and blood were extracted while sacrificing the mice.

Weight gain in mice subjected to treatment with nanosystems Au@16-Ph-16-miR-21 and Au@16-3-16-miR-21 was halted in comparison with controls; particularly, treatment with nanosystem 16-ph-16-miR-21, both with 0.2 µg and with 0.3 µg of miR-21, significantly reduced weight gain in mice subjected to the fat rich diet in comparison with the control (Figure 10).

The study was complemented with a histological analysis and a gene and protein expression analysis in inguinal subcutaneous, interscapular and visceral white adipose tissue and interscapular brown fat originating from mice subjected to *in vivo* treatment with nanosystems Au@16-Ph-16-miR-21 and Au@16-3-16-miR-21.

On one hand, the histological images of the tissues, which were prepared with hematoxylin and eosin staining (Figure 11), showed that *in vivo* treatment with nanosystems Au@16-Ph-16-miR-21 and Au@16-3-16-miR-21 has transformed part of white fat (made up of unilocular adipocytes) into beige fat (made up of multilocular adipocytes).

On the other hand, a gene expression analysis was performed with messenger RNA obtained from inguinal subcutaneous and interscapular adipose tissues, thermogenesis marker genes (UCP1 and PGC-1a), beige cell markers (Tmem26), and browning process regulating genes Vegf-A and Prdm16 by means of real-time PCR (Figure 12). The data demonstrates that both thermogenesis and browning are significantly induced by means of treatment with 0.2 ug of nanosystem Au@16-Ph-16-miR-21.

A protein expression analysis was then performed by means of immunohistochemistry on inguinal subcutaneous white adipose tissue and visceral white adipose tissue with anti-UCP-1 protein antibodies (as a brown adipose tissue marker) and anti-TMEM26 antibodies (as a beige adipocyte marker) alone and in combination (merge) (Figure 13). The immunohistochemistry images show the appearance of a strong signal in the inguinal subcutaneous white fat corresponding to UCP-1 protein and TMEM26 with treatment with nanosystem-miR-21 in comparison with the control (without miR-21). Furthermore, a double labelling with both antibodies revealed the co-localisation of adipocytes with a high expression of UCP-1 and TMEM26 only in the inguinal subcutaneous white fat from mice treated with nanosystem-miR-21 in comparison with the control (without miR-21).

The number of mitochondria in inguinal adipose tissue was also evaluated by means of electron microscope imaging (Figure 14). These images show the scarcity of mitochondria in the subcutaneous white fat (inguinal) of control mice in comparison with an abundance of large, electron-dense organelles in the subcutaneous white fat (inguinal) of mice treated with nanosystem Au@16-pH-16-miR-21.

The data confirms that the effect of nanosystems Au@16-pH-16-miR-21 and Au@16-3-16-miR-21 (at a low concentration of miR-21 of 0.2 and 0.3 µg) on the reduction in weight gain would be mediated by the induction of browning in subcutaneous white adipose tissue and the activation of thermogenesis in both brown fat and subcutaneous white fat by means of the conversion of white adipocytes into beige adipocytes.

## Claims

1. A functionalised nanosystem for transporting biologically active molecules comprising:
(a) a biologically active molecule which is selected from the miR-21 microRNA, mimetic, isomiR, oligonucleotide molecule with more than 75% sequence similarity, compounds aimed at increasing the transcription or activity of miR-21 or a source thereof, or any of the combinations thereof; and
(b) a pharmaceutically acceptable carrier comprising a nanoparticle and a transporter molecule capable of linking oligonucleotides.

2. The nanosystem according to the preceding claim, wherein the biologically active molecule and the nanoparticle are bound to the transporter molecule independently. Both bonds can be either a covalent bond or a non-covalent bond. Generally, by means of electrostatic interactions, hydrophobic interactions, surface adsorption, encapsulation or intercalated inside same.

3. The nanosystem according to any of the preceding claims, wherein the biologically active molecule and the nanoparticle are bound to the transporter molecule independently by means of non-covalent bond which can be: electrostatic interactions, hydrophobic interactions, surface adsorption, encapsulation or intercalated inside same.

4. The nanosystem according to any of the preceding claims, wherein the nanoparticle comprises a cationic metal core.

5. The nanosystem according to any of the preceding claims, wherein the cationic metal core is a cationic gold core.

6. The nanosystem according to any of the preceding claims, wherein the mean size of the nanoparticles is comprised between 1 and 20 nanometres.

7. The nanosystem according to claim 6, wherein the mean size of the nanoparticles is comprised between 3 and 7 nanometres and/or the mean size of the nanosystem is comprised between 30 and 80 nanometres.

8. The nanosystem according to any of the preceding claims, wherein the transporter molecule of the nanosystem is a cationic surfactant.

9. The nanosystem according to any of the preceding claims, wherein the transporter molecule is a Gemini cationic surfactant.

10. The nanosystem according to claim 9, wherein the Gemini surfactant is 16-3-16.

11. The nanosystem according to claim 10, wherein the mean size of the nanoparticle is comprised between 3 and 4 nanometres and/or the mean size of the nanosystem is comprised between 30 and 80 nanometres.

12. The nanosystem according to claim 9, wherein the Gemini surfactant is 16-Ph-16.

13. The nanosystem according to any of claims 12, wherein the mean size of the nanoparticle is comprised between 5 and 6 nanometres and/or the mean size of the nanosystem is comprised between 40 and 60 nanometres.

14. A composition comprising at least one nanosystem according to claims 1-13.

15. The composition according to the preceding claim, which is a pharmaceutical composition.

16. The nanosystem according to any of claims 1-13 or a composition according to claims 14-15, for use as a medicinal product.

17. The nanosystem or composition according to the preceding claim for preventing, delaying, mitigating, reversing, curing and/or treating a metabolic disease; wherein the metabolic disease is selected from the list consisting of: amyloidosis, cardiometabolic disease, dehydration, diabetes (type 1, type 2, diabetic foot ulcers, diabetic macular oedema, diabetic neuropathy, diabetic retinopathy, diabetic nephropathy, gestational diabetes, dyslipidaemia, hyperlipidaemia), glucose intolerance, hypercholesterolaemia, hyperglycaemia, hyperinsulinaemia, or insulin resistance, hyperkalaemia, hypoglycaemia, hypopotassaemia, lipodystrophy (lipoatrophy), metabolic syndrome, obesity, osteopenia, osteoporosis (including postmenopausal osteoporosis), phenylketonuria (PKU), hypersecretion of pituitary ACTH (Cushing's syndrome) and Pompe disease.

18. The nanosystem or composition according to the preceding claim, wherein the metabolic disease is selected from obesity, overweight and hyperinsulinaemia, or insulin resistance.

19. The nanosystem according to any of claims 1-13 or the composition according to claims 14-15 for preventing, delaying, mitigating, reversing, curing and/or treating a disease associated with body weight gain. More preferably, the disease associated with body weight gain is selected from the list consisting of hypothyroidism, Cushing's syndrome, hypogonadism, hypothalamic lesions, growth hormone deficiency, Prader-Willi syndrome, Bardet-Biedl syndrome, Cohen syndrome, MOMO syndrome, anxiety and depression; or any of the combinations thereof.

20. A kit-of-parts comprising:
(a) a nanosystem according to claims 1-13 or a composition according to claims 14-15; and (b) a medicinal product which is selected from:
(b1) a medicinal product associated with weight gain which is selected from the list consisting of: atenolol, carbamazepine, citalopram, clozapine, doxazosin mesylate, doxepin, escitalopram, fluvoxamine, gabapentin, gamma-hydroxybutyric acid, leuprolide, lithium, metoprolol, mirtazapine, nateglinide, nortriptyline, olanzapine, paroxetine, pioglitazone, propranolol, quetiapine, repaglinide, risperidone, terazosin, valproate and phenytoin; or
(b2) a medicinal product for obesity approved in a national agency which is selected from the list consisting of: megestrol acetate, benzphetamine, caffeine, cathinone, cetilistat, clobenzorex, chlorphentermine hydrochloride, dexfenfluramine hydrochloride, diethylpropion hydrochloride, fenfluramine hydrochloride, phenmetrazine hydrochloride, phentermine hydrochloride, lorcaserin hydrochloride, mefenorex hydrochloride, sibutramine hydrochloride, dronabinol, phendimetrazine, fenfluramine, phenylpropanolamine, fenproporex, phentermine, fluoxetine, levocarnitine, levothyroxine sodium, mazindol, methamphetamine, methylcellulose, orlistat, phendimetrazine, rimonabant, Saxenda, sibutramine, amphetamine sulfate, phendimetrazine tartrate, "bupropion hydrochloride + naltrexone", "phentermine hydrochloride + topiramate", "levocarnitine + sibutramine" and "metformin + sibutramine"; or
(b3) a medicinal product for obesity in the clinical development phase which is selected from the list consisting of: Adipotide, AKR-001, AM-833, AMG-598, beloranib, BI-456906, biotin, betahistine hydrochloride, lorcaserin hydrochloride, lorcaserin hydrochloride, efpeglenatide, G-3215, GMA-102, GT-001, GTS-21, HM-12525A, HM-15211, HSG-4112, LLF-580, MEDI-0382, MET-2, Miricorilant, NGM-313, NGM-386, NN-9277, NN-9423, NN-9536, NNC-01651562, NNC-01651875, Novdb-2, NovOB, pegapamodutide, REGN-4461, RZL-12, S-237648, S-237648, SAR-425899, SCO-792, setmelanotide, setmelanotide, tesofensine, TP-0101, VP-01, ZGN-1061, ZP-4982, (acarbose + orlistat), (leucine + sildenafil citrate), (leucine + metformin hydrochloride + sildenafil citrate) and (metoprolol + tesofensine).

21. The kit-of-parts according to the preceding claim for preventing, delaying, mitigating, reversing, curing and/or treating a metabolic disease, wherein the metabolic disease is selected from the list consisting of: amyloidosis, cardiometabolic disease, dehydration, diabetes (type 1, type 2, diabetic foot ulcers, diabetic macular oedema, diabetic neuropathy, diabetic retinopathy, diabetic nephropathy, gestational diabetes, dyslipidaemia, hyperlipidaemia), glucose intolerance, hypercholesterolaemia, hyperglycaemia, hyperinsulinaemia, or insulin resistance, hyperkalaemia, hypoglycaemia, hypopotassaemia, lipodystrophy (lipoatrophy), metabolic syndrome, obesity, osteopenia, osteoporosis (including postmenopausal osteoporosis), phenylketonuria (PKU), hypersecretion of pituitary ACTH (Cushing's syndrome) and Pompe disease.

22. The kit-of-parts according to claim 21, wherein the metabolic disease is selected from obesity, overweight and hyperinsulinaemia, or insulin resistance.

23. A method for synthesising a nanosystem according to claims 1-13 which comprises:
(a) adding a Gemini surfactant as a stabilising agent in a hydrogen tetrachloroaurate tetrahydrate solution (HAuCl₄•4H₂O),
(b) reducing hydrogen tetrachloroaurate tetrahydrate (HAuCl₄•4H₂O) by means of the controlled addition of a reducing agent, and
(c) complexing with the biologically active molecule by means of the controlled addition of the miR-21 microRNA, mimetic, isomiR, oligonucleotide molecule with more than 75% sequence similarity, compounds aimed at increasing the transcription or activity of miR-21 or a source thereof or any of the combinations thereof; with excess polymer and stirring conditions being maintained.

24. The method according to the preceding claim, wherein the reducing agent is sodium borohydride (NaBH₄).

25. The method according to any of claims 23-24, wherein the reducing agent is metered by means of fractionated dropwise addition.

26. The method according to any of claims 23-25, wherein vigorous stirring is performed between step (a) and step (b) in the absence of light for at least 3 minutes.

27. The method according to the preceding claim, wherein the vigorous stirring in the absence of light is performed for at least 4 minutes.

28. The method according to any of claims 23-27, wherein stirring is performed between step (b) and step (c) in the absence of light for at least 10 minutes.

29. The method according to the preceding claim, wherein the stirring in the absence of light is performed for at least 12 minutes.

30. The method according to any of claims 23-28, wherein gentle stirring is performed after step (c) for at least 20 minutes.

31. The method according to the preceding claim, wherein the gentle stirring is performed for at least 25 minutes.
